# EUROPEAN PATENT APPLICATION

(11) **EP 4 834 358 A1**
(43) Date of publication of application: **04.11.2026**
(21) Application number: 24913025.3
(22) Date of filing: 26.12.2024
(51) Int. Cl.: A61K 38/10, A61K 49/00, A61P 21/04, A61P 37/02, A61P 37/06, A61P 43/00, C07K 14/705, C12Q 1/04

(54) **AGENT CONTAINING POLYPEPTIDE COMPRISING AMINO ACID SEQUENCE CORRESPONDING TO HLA-DR-BINDING EPITOPE OF ACHR**

(30) Priority: 28.12.2023 JP 2023222935
(71) Applicant: Denka Company Limited, Tokyo 103-8338 (JP)
(72) Inventor: FUJIMORI, Satoshi, Tokyo 103-8338 (JP); FUKUSHIMA, Naoshi, Tokyo 103-8338 (JP); KUBO, Hiroshi, Tokyo 103-8338 (JP); MIYASHITA, Yohei, Tokyo 103-8338 (JP); OKADA, Hiromasa, Tokyo 103-8338 (JP); ONOZUKA, Wataru, Tokyo 103-8338 (JP); NISHIO, Marie, Tokyo 103-8338 (JP)
(74) Representative: Müller-Boré & Partner Patentanwälte PartG mbB
(86) International application number: PCT/JP2024/046203
(87) International publication number: WO 2025/143139

(57) **Abstract**

Disclosed is an activating agent for regulatory T cells, an activity inhibitory agent for effector T cells, an activation inhibitory agent for B cells, a suppressive agent for humoral immunity, or a therapeutic agent or a preventive agent for an autoimmune disease, comprising a polypeptide including an amino acid sequence corresponding to an HLA-DR-binding epitope of AChR.

## Description

### TECHNICAL FIELD

The present disclosure relates to an agent containing a polypeptide comprising an amino acid sequence corresponding to an HLA-DR-binding epitope of AChR.

### BACKGROUND

In autoimmune diseases, the immune system, which is ordinarily a system for removing foreign matter originating outside an organism, mistakenly identifies matter originating inside the organism as an antigen, thereby causing abnormal immune activation. Such abnormal immune activation is known to cause various abnormalities in biological functions inside the bodies of patients suffering from autoimmune diseases.

Early-onset myasthenia gravis is a form of myasthenia gravis that occurs in subjects aged 10 to 49. Myasthenia gravis, designated as one of the intractable diseases in Japan, is an organ-specific autoimmune disease directed against molecules on the postsynaptic membrane at the neuromuscular junction, having reduced muscular strength as the main symptom.

In myasthenia gravis, autoantibodies are created in a patient's body against acetylcholine receptor (AChR) and muscle-specific receptor tyrosine kinase (MuSK), which are molecules present on the postsynaptic membrane at the neuromuscular junction. These antibodies cause various symptoms by inhibiting neurotransmission. Approximately 80% of myasthenia gravis cases are known to be caused by autoantibodies to AChR.

In normal humoral immunity, antigen-presenting cells present antigens to naive CD4⁺ T cells, causing the naive CD4⁺ T cells to differentiate into effector CD4⁺ T cells. This activates B cells and antibody production occurs. However, in recent years, as a new type of antigen-presenting cells, the existence of antigen-presenting cells that have the function of presenting antigens to naive CD4⁺ T cells and thereby causing the naive CD4⁺ T cells to differentiate not into effector CD4⁺ T cells, but rather into regulatory T cells, has been reported (Non-Patent Documents 1 and 2). Such antigen-presenting cells, by presenting antigens to activate regulatory T cells, inhibit the activation of B cells by effector CD4⁺ T cells presented with the same antigen. They therefore have the function of suppressing antibody production, contrary to the functions of antigen-presenting cells that were previously known. In fact, clinical trials have been performed for autoimmune diseases using antibody production-suppressing agents targeting these new types of antigen-presenting cells (Non-Patent Document 3).

In autoimmune diseases, human leukocyte antigens (HLAs), which are protein complexes that serve the role of presenting antigens on the surfaces of antigen-presenting cells, recognize portions of the antigens and form bonds with the antigens. In particular, when the antigen is a protein, the sites on an antigen protein that are recognized by HLAs are called epitopes. Since such epitopes have the potential to serve as targets for treatment and diagnosis of autoimmune diseases, there have been attempts to identify them in various autoimmune diseases. For example, the epitopes in early-onset myasthenia gravis have also been reported (Non-Patent Document 4).

### CITATION LIST

### PATENT LITERATURE

Non-Patent Document 1: Laura Passerini and Silvia Gregori, "Induction of Antigen-Specific Tolerance in T Cell Mediated Diseases", Frontiers in Immunology, 11(2194) (2020)
Non-Patent Document 2: Matthias G. von Herrath and Leonard C. Harrison, "Antigen-induced Regulatory T Cells in Autoimmunity", Nature Review Immunology, 3: 223-232 (2003)
Non-Patent Document 3: Ciaran P. Kelly et al., "TAK-101 Nanoparticles Induce Gluten-Specific Tolerance in Celiac Disease: A Randomized, Double-Blind, Placebo-Controlled Study", Gastroenterology, 161(1): 66-80.e8 (2021)
Non-Patent Document 4: Takayuki Kanai et al., "Immuno-suppressive peptides for a human T cell clone autoreactive to a unique acetylcholine receptor alpha subunit peptide presented by the disease-susceptible HLA-DQ6 in infant-onset myasthenia gravis", Hum. Immunol., 56(1-2): 28-38 (1997)

### SUMMARY OF INVENTION

### TECHNICAL PROBLEM

An objective of the present invention is to provide an agent that contains a polypeptide including an amino acid sequence corresponding to an epitope of AChR, and that can potentially be applied to the treatment or diagnosis of an autoimmune disease.

### SOLUTION TO PROBLEM

The present inventors, by means of a NAPA (natural antigen processing assay) method using dendritic cells prepared from classical monocytes from humans, discovered AChR peptide fragments that are secreted as complexes with human leukocyte antigens (HLAs), from human dendritic cells that have taken in AChR. Furthermore, the present inventors discovered that these peptide fragments include amino acid sequences corresponding to epitopes of AChR, and that agents containing polypeptides including said amino acid sequences can potentially be applied to the treatment or diagnosis of autoimmune diseases.

The present disclosure, for example, relates to the following.
[1] An activating agent or a composition for activation of regulatory T cells, containing at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[2] An activity inhibitory agent or a composition for inhibiting activity of effector T cells, containing at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[3] An activation inhibitory agent or a composition for inhibiting activation of B cells, containing at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[4] A suppressive agent or a composition for suppression of humoral immunity, containing at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[5] A therapeutic agent, a preventive agent, a composition for treatment, or a composition for prevention of an autoimmune disease, containing at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[6] A treatment method or a prevention method for an autoimmune disease, wherein the method includes administering, to a subject in need thereof, at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[7] At least one polypeptide for use in treating or preventing an autoimmune disease, wherein the polypeptide is selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[8] Use of at least one polypeptide in production of a therapeutic agent, a preventive agent, a composition for treatment, or a composition for prevention of an autoimmune disease, wherein the polypeptide is selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[9] At least one polypeptide for use in production of a therapeutic agent, a preventive agent, a composition for treatment, or a composition for prevention of an autoimmune disease, wherein the polypeptide is selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[10] A kit for diagnosing an autoimmune disease, containing at least one polypeptide and a human leukocyte antigen that binds to the at least one polypeptide, wherein the at least one polypeptide is selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[11] The kit according to [10], further containing a labeling dye that binds to the human leukocyte antigen.
[12] A diagnostic agent for an autoimmune disease, containing a complex comprising a polypeptide, a human leukocyte antigen, and a labeling dye, wherein the polypeptide is at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[13] A method for aiding in diagnosis of an autoimmune disease, wherein the method includes detecting T cells having antigen specificity to at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[14] A method for acquiring data for diagnosis of an autoimmune disease, wherein the method includes detecting T cells having antigen specificity to at least one polypeptide selected from the group consisting of:
   (A) one or more polypeptides having a full length of at least 8 residues and at most 20 residues, including an amino acid sequence having 90% or higher sequence identity with an amino acid sequence according to SEQ ID NO: 1;
   (B) one or more polypeptides having a full length of at least 12 residues and at most 21 residues, including an amino acid sequence having 90% or higher sequence identity with an amino acid sequence according to SEQ ID NO: 2;
   (D) one or more polypeptides having a full length of at least 7 residues and at most 17 residues, including an amino acid sequence having 90% or higher sequence identity with an amino acid sequence according to SEQ ID NO: 4; and
   (E) one or more polypeptides having a full length of at least 10 residues and at most 19 residues, including an amino acid sequence having 90% or higher sequence identity with an amino acid sequence according to SEQ ID NO: 5.
[15] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1.
[16] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (B) one or more polypeptides having a full length of at least 12 residues and at most 21 residues, including an amino acid sequence having 90% or higher sequence identity with the amino acid sequence according to SEQ ID NO: 2.
[17] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (D) one or more polypeptides having a full length of at least 7 residues and at most 17 residues, including an amino acid sequence having 90% or higher sequence identity with the amino acid sequence according to SEQ ID NO: 4.
[18] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (E) one or more polypeptides having a full length of at least 10 residues and at most 19 residues, including an amino acid sequence having 90% or higher sequence identity with the amino acid sequence according to SEQ ID NO: 5.
[19] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ **ID** NO: 1; and
   (B) one or more polypeptides having a full length of at least 12 residues and at most 21 residues, including an amino acid sequence having 90% or higher sequence identity with the amino acid sequence according to SEQ **ID** NO: 2.
[20] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1; and
   (D) one or more polypeptides having a full length of at least 7 residues and at most 17 residues, including an amino acid sequence having 90% or higher sequence identity with the amino acid sequence according to SEQ ID NO: 4.
[21] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1; and
   (E) one or more polypeptides having a full length of at least 10 residues and at most 19 residues, including an amino acid sequence having 90% or higher sequence identity with the amino acid sequence according to SEQ ID NO: 5.
[22] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (B) one or more polypeptides having a full length of at least 12 residues and at most 21 residues, including an amino acid sequence having 90% or higher sequence identity with the amino acid sequence according to SEQ ID NO: 2; and
   (D) one or more polypeptides having a full length of at least 7 residues and at most 17 residues, including an amino acid sequence having 90% or higher sequence identity with the amino acid sequence according to SEQ ID NO: 4.
[23] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (B) one or more polypeptides having a full length of at least 12 residues and at most 21 residues, including an amino acid sequence having 90% or higher sequence identity with the amino acid sequence according to SEQ ID NO: 2; and
   (E) one or more polypeptides having a full length of at least 10 residues and at most 19 residues, including an amino acid sequence having 90% or higher sequence identity with the amino acid sequence according to SEQ ID NO: 5.
[24] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (D) one or more polypeptides having a full length of at least 7 residues and at most 17 residues, including an amino acid sequence having 90% or higher sequence identity with the amino acid sequence according to SEQ ID NO: 4; and
   (E) one or more polypeptides having a full length of at least 10 residues and at most 19 residues, including an amino acid sequence having 90% or higher sequence identity with the amino acid sequence according to SEQ ID NO: 5.
[25] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1;
   (B) one or more polypeptides having a full length of at least 12 residues and at most 21 residues, including an amino acid sequence having 90% or higher sequence identity with the amino acid sequence according to SEQ ID NO: 2; and
   (D) one or more polypeptides having a full length of at least 7 residues and at most 17 residues, including an amino acid sequence having 90% or higher sequence identity with the amino acid sequence according to SEQ ID NO: 4.
[26] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1;
   (B) one or more polypeptides having a full length of at least 12 residues and at most 21 residues, including an amino acid sequence having 90% or higher sequence identity with the amino acid sequence according to SEQ ID NO: 2; and
   (E) one or more polypeptides having a full length of at least 10 residues and at most 19 residues, including an amino acid sequence having 90% or higher sequence identity with the amino acid sequence according to SEQ ID NO: 5.
[27] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1;
   (D) one or more polypeptides having a full length of at least 7 residues and at most 17 residues, including an amino acid sequence having 90% or higher sequence identity with the amino acid sequence according to SEQ ID NO: 4; and
   (E) one or more polypeptides having a full length of at least 10 residues and at most 19 residues, including an amino acid sequence having 90% or higher sequence identity with the amino acid sequence according to SEQ ID NO: 5.
[28] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (B) one or more polypeptides having a full length of at least 12 residues and at most 21 residues, including an amino acid sequence having 90% or higher sequence identity with the amino acid sequence according to SEQ ID NO: 2;
   (D) one or more polypeptides having a full length of at least 7 residues and at most 17 residues, including an amino acid sequence having 90% or higher sequence identity with the amino acid sequence according to SEQ ID NO: 4; and
   (E) one or more polypeptides having a full length of at least 10 residues and at most 19 residues, including an amino acid sequence having 90% or higher sequence identity with the amino acid sequence according to SEQ ID NO: 5.
[29] The activating agent or the composition according to [1], the activity inhibitory agent or the composition according to [2], the activation inhibitory agent or the composition according to [3], the suppressive agent or the composition according to [4], the therapeutic agent, the preventive agent, or the composition according to [5], the method according to [6], the at least one polypeptide according to [7], the use according to [8], the at least one polypeptide according to [9], the kit according to [10] or [11], the diagnostic agent according to [12], or the method according to [13] or [14], wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1;
   (B) one or more polypeptides having a full length of at least 12 residues and at most 21 residues, including an amino acid sequence having 90% or higher sequence identity with the amino acid sequence according to SEQ ID NO: 2;
   (D) one or more polypeptides having a full length of at least 7 residues and at most 17 residues, including an amino acid sequence having 90% or higher sequence identity with the amino acid sequence according to SEQ ID NO: 4; and
   (E) one or more polypeptides having a full length of at least 10 residues and at most 19 residues, including an amino acid sequence having 90% or higher sequence identity with the amino acid sequence according to SEQ ID NO: 5.
[30] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [29], for use in a subject in whom an HLA-DRB3 genotype is 01:01 and/or an HLA-DRB1 genotype is 03:01, wherein the at least one polypeptide includes:
   (A) one or more polypeptides having a full length of at least 8 residues and at most 20 residues, including an amino acid sequence having 90% or higher sequence identity with the amino acid sequence according to SEQ ID NO: 1.
[31] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [30], for use in a subject in whom an HLA-DRB3 genotype is 01:01, wherein the at least one polypeptide includes:
   (B) one or more polypeptides having a full length of at least 12 residues and at most 21 residues, including an amino acid sequence having 90% or higher sequence identity with the amino acid sequence according to SEQ ID NO: 2.
[32] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [31], for use in a subject in whom an HLA-DRB3 genotype is 01:01 and/or an HLA-DRB1 genotype is 09:01, wherein the at least one polypeptide includes:
   (D) one or more polypeptides having a full length of at least 7 residues and at most 17 residues, including an amino acid sequence having 90% or higher sequence identity with the amino acid sequence according to SEQ ID NO: 4.
[33] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [32], for use in a subject in whom an HLA-DRB3 genotype is 01:01 and/or an HLA-DRB1 genotype is 03:01, wherein the at least one polypeptide includes:
   (E) one or more polypeptides having a full length of at least 10 residues and at most 19 residues, including an amino acid sequence having 90% or higher sequence identity with the amino acid sequence according to SEQ ID NO: 5.
[34] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [33], wherein the (A) one or more polypeptides having a full length of at least 8 residues and at most 20 residues, including an amino acid sequence having 90% or higher sequence identity with the amino acid sequence according to SEQ ID NO: 1 are one or more polypeptides having 90% or higher sequence identity with an amino acid sequence selected from the group consisting of the amino acid sequences according to SEQ ID NOs: 6-15 (A').
[35] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [34], wherein the (B) one or more polypeptides having a full length of at least 12 residues and at most 21 residues, including an amino acid sequence having 90% or higher sequence identity with the amino acid sequence according to SEQ ID NO: 2 are (B') one or more polypeptides having 90% or higher sequence identity with the amino acid sequence according to SEQ ID NO: 16.
[36] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [35], wherein the (D) one or more polypeptides having a full length of at least 7 residues and at most 17 residues, including an amino acid sequence having 90% or higher sequence identity with the amino acid sequence according to SEQ ID NO: 4 is (D') one or more polypeptides having 90% or higher sequence identity with an amino acid sequence selected from the group consisting of the amino acid sequences according to SEQ ID NOs: 22-32.
[37] The activating agent, the composition, the activity inhibitory agent, the activation inhibitory agent, the suppressive agent, the therapeutic agent, the preventive agent, the method, the at least one polypeptide, the use, the kit, or the diagnostic agent according to any one of [1] to [36], wherein the (E) one or more polypeptides having a full length of at least 10 residues and at most 19 residues, including an amino acid sequence having 90% or higher sequence identity with the amino acid sequence according to SEQ ID NO: 5 is (E') one or more polypeptides having 90% or higher sequence identity with an amino acid sequence selected from the group consisting of the amino acid sequences according to SEQ ID NOs: 33-35.
[38] The therapeutic agent, the preventive agent, the composition, the method, the at least one polypeptide, the use, the kit, or the diagnostic agent according to any one of [5] to [37], wherein the autoimmune disease is myasthenia gravis.
[39] The therapeutic agent, the preventive agent, the composition, the method, the at least one polypeptide, the use, the kit, or the diagnostic agent according to any one of [5] to [38], wherein the autoimmune disease is early-onset myasthenia gravis.

### EFFECTS OF INVENTION

The present disclosure provides an agent that contains a polypeptide including an amino acid sequence corresponding to an epitope of AChR, and that can potentially be applied to the treatment or diagnosis of an autoimmune disease.

The polypeptide including an amino acid sequence corresponding to an epitope of AChR that is contained in the agent according to the present disclosure was discovered by means of a NAPA method using dendritic cells prepared from classical monocytes from humans and is highly likely to be actually present in the body of a patient suffering from an autoimmune disease. Therefore, it has the potential to be more suitable for application to the treatment or diagnosis of autoimmune diseases than polypeptides with sequences corresponding to epitopes that have previously been discovered.

### BRIEF DESCRIPTION OF DRAWINGS

FIG. 1 is a diagram indicating, as coordinates on human AChRα1, HLA-binding peptides identified in Example 1.
FIG. 2 is a diagram indicating the results from ICS (upper part) and ELISA (lower part) obtained by performing six independent specific T cell inducibility evaluation tests for each peptide using PBMCs from healthy donors in Example 2.
FIG. 3 is a diagram indicating the results from ELISA analyses for evaluating the presence or absence of Th1 cells specific to SEQ ID NO: 17 using five PBMC specimens from an MG patient having DRB3*01:01, which is a predicted restriction allele in SEQ ID NO: 17 in Example 3.
FIG. 4 is a diagram indicating the results from ICS (left side) and ELISA (right side) when evaluating Th1 cell inducibility specific to SEQ ID NO: 17 using PBMCs from healthy donors having HLA-DRB1*09:01, which is reported to be associated with MG in Asians, and not having DRB1*03:01 and DRB3*01:01.

### DESCRIPTION OF EMBODIMENTS

Hereinafter, modes for carrying out the present disclosure will be explained. However, the present disclosure is not limited to the embodiments below.

### <Sequence identity>

In the present disclosure, "sequence identity" refers to the percentage (%) of residues that are identical to the residues in overlapping amino acid sequences in an optimal alignment when two amino acid sequences are aligned. In the present disclosure, mutations in amino acid sequences for which the sequence identity with respect to a certain amino acid sequence is not 100% may be, for example, substitutions, deletions, or insertions of amino acid residues. More specifically, they may be, for example, single residue substitutions, single residue deletions, or single residue insertions, and these deletions or insertions may occur at an end of an amino acid sequence or may occur somewhere other than an end.

### <Human leukocyte antigen>

Human leukocyte antigens (HLAs) are major histocompatibility complexes (MHCs) in humans. In humoral immunity, HLAs are exposed on the cell surfaces of antigen-presenting cells (APCs) in a state in which antigens are bound thereto, thereby driving an antibody production mechanism with respect to those antigens. HLAs are classified into HLA class I, to which HLA-A, HLA-C, HLA-B, etc. belong and which is involved in cellular immunity, and HLA class II, to which HLA-DR, HLA-DQ, HLA-DP, etc. belong and which is involved in humoral immunity.

Due to HLA genotypes, human leukocytes include several tens of thousands of possible genotypes. The genotype of HLA is one of the risk factors for autoimmune diseases, and it is known that there is a high probability of occurrence of autoimmune diseases in humans having specific HLA genotypes. In particular, having the 01:01 genotype for HLA-DRB3 or having the 03:01 genotype for HLA-DRB1 is known to be a risk factor for early-onset myasthenia gravis.

According to the notation method defined by the WHO Nomenclature Committee for Factors of the HLA System, the genotypes (alleles) of HLA are notated in the following form: "HLA-(gene symbol)*(first field):(second field):(third field):(fourth field)". In this case, the first field to the fourth field are each represented by a two-digit number, the first field representing the HLA specificity (antigenic type), the second field representing non-synonymous substitutions (variations in the amino acid sequence in the same antigenic type), the third field representing synonymous substitutions (variations in the exon region that do not cause changes in the coded amino acid sequence), and the fourth field representing base substitutions outside the coding region (variations in the sequence in the intron region). In this notation, the gene symbol, the first field, and the second field represent information that directly affects the amino acid sequence of the HLA, and are thus clinically important.

In the present disclosure, the first field being YY and the second field being ZZ in the genotype of the HLA gene for which the gene symbol is XXX (i.e., where the nomenclature up to the second field in the above-mentioned notation method is represented by HLA-XXX*YY:ZZ) will also be described as "the genotype of HLA-XXX is YY:ZZ". That is, for example, "the genotype of HLA-DRB3 is 01:01" means that, in the genotype of the HLA gene for which the gene symbol is DRB3 (the HLA-DRB3 gene), the first field is 01 and the second field is 01, i.e., the genotype is HLA-DRB3*01:01. Additionally, for example, "the genotype of HLA-DRB1 is 03:01" means that, in the genotype of the HLA gene for which the gene symbol is DRB1 (the HLA-DRB1 gene), the first field is 03 and the second field is 01, i.e., the genotype is HLA-DRB1*03:01.

### <Acetylcholine receptor>

Acetylcholine receptors (AChR) are receptors having a function that is indispensable for transmitting information between nerve cells by the binding of extracellularly secreted acetylcholine in neurotransmission. In an autoimmune disease in which antibodies to acetylcholine receptors are overproduced, some of the neurotransmission mediated by acetylcholine receptors is inhibited, causing various symptoms. Additionally, among acetylcholine receptors, the α1 subunit in human acetylcholine receptor (human AChRα1), in particular, is known to be recognized as an autoantigen in approximately 80% of early-onset myasthenia gravis patients. Human AChRα1 has the amino acid sequence (SEQ ID NO: 36) of RefSeq accession no. NP_000070.1 at the National Center for Biotechnology Information (NCBI) in the United States.

### <Activating agent or composition for activation of regulatory T cells>

A first aspect of the present disclosure relates to an activating agent for regulatory T cells, containing at least one polypeptide selected from the group consisting of (A), (B), (D), and (E) below:
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2;
(D) one or more polypeptides having a full length of at least 7 residues and at most 17 residues, including an amino acid sequence having 90% or higher sequence identity with an amino acid sequence according to SEQ ID NO: 4; and
(E) one or more polypeptides having a full length of at least 10 residues and at most 19 residues, including an amino acid sequence having 90% or higher sequence identity with an amino acid sequence according to SEQ ID NO: 5. The first aspect of the present disclosure may, in one embodiment, be a composition for activation of regulatory T cells containing the above-mentioned at least one polypeptide.

The agent according to one embodiment may contain the above-mentioned (A). The agent according to one embodiment may contain the above-mentioned (B). The agent according to one embodiment may contain the above-mentioned (D). The agent according to one embodiment may contain the above-mentioned (E). The agent according to one embodiment may contain the above-mentioned (A) and the above-mentioned (B). The agent according to one embodiment may contain the above-mentioned (A) and the above-mentioned (D). The agent according to one embodiment may contain the above-mentioned (A) and the above-mentioned (E). The agent according to one embodiment may contain the above-mentioned (B) and the above-mentioned (D). The agent according to one embodiment may contain the above-mentioned (B) and the above-mentioned (E). The agent according to one embodiment may contain the above-mentioned (D) and the above-mentioned (E). The agent according to one embodiment may contain the above-mentioned (A), the above-mentioned (B), and the above-mentioned (D). The agent according to one embodiment may contain the above-mentioned (A), the above-mentioned (B), and the above-mentioned (E). The agent according to one embodiment may contain the above-mentioned (A), the above-mentioned (D), and the above-mentioned (E). The agent according to one embodiment may contain the above-mentioned (B), the above-mentioned (D), and the above-mentioned (E). The agent according to one embodiment may contain the above-mentioned (A), the above-mentioned (B), the above-mentioned (D), and the above-mentioned (E).

When one or more polypeptides represented by the above-mentioned (A), (B), (D), and (E) include an amino acid sequence having 90% or higher sequence identity with a prescribed sequence (for example, the sequences represented by SEQ ID NOs: 1-17 and 22-35, or a portion of the sequence represented by SEQ ID NO: 37) or comprise said amino acid sequence, the sequence identity of said amino acid sequence with respect to said prescribed sequence may be higher than 90%. For example, the sequence identity may be 91% or higher, 92% or higher, 93% or higher, 94% or higher, 95% or higher, 96% or higher, 97% or higher, 98% or higher, 99% or higher, or 100%. Additionally, when one or more polypeptides represented by the above-mentioned (A), (B), (D), and (E) include an amino acid sequence having 90% or higher sequence identity with a prescribed sequence (for example, the sequences represented by SEQ ID NOs: 1-17 and 22-35, or a portion of the sequence represented by SEQ ID NO: 37) or comprise said amino acid sequence, said amino acid sequence may be an amino acid sequence including, for example, said prescribed sequence or an amino acid sequence including a mutation at one residue, at two residues, or at three residues in said prescribed sequence, and in one preferable embodiment, may be said prescribed sequence or an amino acid sequence including a mutation at one residue in said prescribed sequence.

The amino acid sequence according to SEQ ID NO: 1 corresponds to the 121-st to 131-st positions in the amino acid sequence (SEQ ID NO: 36) of the α1 subunit of human acetylcholine receptor, having RefSeq accession no. NP_000070.1 at the National Center for Biotechnology Information (NCBI) in the United States. Additionally, the amino acid sequence according to SEQ ID NO: 1 corresponds to the 101-st to 111-th positions in the amino acid sequence (SEQ ID NO: 37) obtained by removing the 1-st to the 20-th positions, which correspond to a signal peptide, from the amino acid sequence indicated by SEQ ID NO: 36.

### SEQ ID NO: 1: AIVKFTKVLLQ

In one embodiment of the present disclosure, the full length of the (A) one or more polypeptides may be, for example, at least 8 residues, at least 9 residues, at least 10 residues, at least 11 residues, at least 12 residues, at least 13 residues, or at least 14 residues, and may be at most 20 residues, at most 19 residues, at most 18 residues, at most 17 residues, at most 16 residues, at most 15 residues, at most 14 residues, at most 13 residues, at most 12 residues, or at most 11 residues. Additionally, in one embodiment of the present disclosure, the full length of the above-mentioned (A) one or more polypeptides may be, for example, at least 8 residues and at most 20 residues, at least 9 residues and at most 19 residues, at least 10 residues and at most 18 residues, at least 11 residues and at most 17 residues, at least 12 residues and at most 17 residues, at least 12 residues and at most 15 residues, at least 12 residues and at most 13 residues, or 12 residues. In one preferable embodiment, the full length of the above-mentioned (A) one or more polypeptides may be at least 12 residues and at most 17 residues.

In one embodiment of the present disclosure, the above-mentioned (A) one or more polypeptides include an amino acid sequence having 90% or higher, 92% or higher, 95% or higher, 98% or higher, or 100% sequence identity with the amino acid sequence according to SEQ ID NO: 1. In one embodiment of the present disclosure, the above-mentioned (A) one or more polypeptides include the amino acid sequence according to SEQ ID NO: 1 or an amino acid sequence having a mutation at one residue, at two residues, or at three residues in said amino acid sequence.

In one embodiment of the present disclosure, the above-mentioned (A) one or more polypeptides may include an amino acid sequence having 90% or higher sequence identity with at least one amino acid sequence selected from the group consisting of the 98-th to 117-th positions, the 99-th to 117-th positions, the 100-th to 117-th positions, the 101-th to 117-th positions, the 98-th to 116-th positions, the 99-th to 116-th positions, the 100-th to 116-th positions, the 101-st to 116-th positions, the 98-th to 115-th positions, the 99-th to 115-th positions, the 100-th to 115-th positions, the 101-th to 115-th positions, the 98-th to 114-th positions, the 99-th to 114-th positions, the 100-th to 114-th positions, the 101-st to 114-th positions, the 98-th to 113-th positions, the 99-th to 113-th positions, the 100-th to 113-th positions, the 101-th to 113-th positions, the 98-th to 112-th positions, the 99-th to 112-th positions, the 100-th to 112-th positions, the 101-st to 112-th positions, the 98-th to 111-th positions, the 99-th to 111-th positions, the 100-th to 111-th positions, and the 101-st to 111-th positions in the amino acid sequence indicated by SEQ ID NO: 37, may comprise an amino acid sequence having 90% or higher sequence identity with said at least one amino acid sequence, or may comprise said at least one amino acid sequence. For example, the 98-th to 117-th positions in the amino acid sequence indicated by SEQ ID NO: 37 correspond to the amino acid sequence indicated by SEQ ID NO: 3. In one embodiment of the present invention, the above-mentioned (A) one or more polypeptides may include an amino acid sequence having 90% or higher sequence identity with the amino acid sequence indicated by SEQ ID NO: 3, may comprise an amino acid sequence having 90% or higher sequence identity with the amino acid sequence indicated by SEQ ID NO: 3, or may comprise said amino acid sequence.

### SEQ ID NO: 3: GDFAIVKFTKVLLQYTGHIT

In one embodiment of the present disclosure, the above-mentioned (A) one or more polypeptides may be one or more polypeptides having 90% or higher sequence identity with an amino acid sequence selected from the group consisting of amino acid sequences according to SEQ ID NOs: 6-15( A'), or may be one or more polypeptides comprising an amino acid sequence selected from the group consisting of amino acid sequences according to SEQ ID NOs: 6-15 (A'). The amino acid sequence indicated by SEQ ID NO: 6 corresponds to the 98-th to 111-th positions in the amino acid sequence indicated by SEQ ID NO: 37. The amino acid sequence indicated by SEQ ID NO: 7 corresponds to the 98-th to 113-th positions in the amino acid sequence indicated by SEQ ID NO: 37. The amino acid sequence indicated by SEQ ID NO: 8 corresponds to the 98-th to 114-th positions in the amino acid sequence indicated by SEQ ID NO: 37. The amino acid sequence indicated by SEQ ID NO: 9 corresponds to the 99-th to 111-th positions in the amino acid sequence indicated by SEQ ID NO: 37. The amino acid sequence indicated by SEQ ID NO: 10 corresponds to the 99-th to 113-th positions in the amino acid sequence indicated by SEQ ID NO: 37. The amino acid sequence indicated by SEQ ID NO: 11 corresponds to the 99-th to 114-th positions in the amino acid sequence indicated by SEQ ID NO: 37. The amino acid sequence indicated by aSEQ ID NO: 12 corresponds to the 100-th to 111-th positions in the amino acid sequence indicated by SEQ ID NO: 37. The amino acid sequence indicated by SEQ ID NO: 13 corresponds to the 100-th to 114-th positions in the amino acid sequence indicated by SEQ ID NO: 37. The amino acid sequence indicated by SEQ ID NO: 14 corresponds to the 101-st to 114-th positions in the amino acid sequence indicated by SEQ ID NO: 37. The amino acid sequence indicated by SEQ ID NO: 15 corresponds to the 101-st to 117-th positions in the amino acid sequence indicated by SEQ ID NO: 37.
SEQ ID NO: 6: GDFAIVKFTKVLLQ
SEQ ID NO: 7: GDFAIVKFTKVLLQYT
SEQ ID NO: 8: GDFAIVKFTKVLLQYTG
SEQ ID NO: 9: DFAIVKFTKVLLQ
SEQ ID NO: 10: DFAIVKFTKVLLQYT
SEQ ID NO: 11: DFAIVKFTKVLLQYTG
SEQ ID NO: 12: FAIVKFTKVLLQ
SEQ ID NO: 13: FAIVKFTKVLLQYTG
SEQ ID NO: 14: AIVKFTKVLLQYTG
SEQ ID NO: 15: AIVKFTKVLLQYTGHIT

The present inventors discovered that the above-mentioned (A) one or more polypeptides bind, among human HLAs, to HLA-DRB3*01:01 and HLA-DRB1*03:01. Therefore, the agent according to one embodiment of the present disclosure may be an agent for use in a subject in whom the HLA-DRB3 genotype is 01:01 and/or the HLA-DRB1 genotype is 03:01, wherein the above-mentioned at least one polypeptide includes the above-mentioned (A) one or more polypeptides.

The amino acid sequence according to SEQ ID NO: 2 corresponds to the 110-th to 124-th positions in the amino acid sequence (SEQ ID NO: 36) of the α1 subunit of human acetylcholine receptor, having RefSeq accession no. NP_000070.1 at the National Center for Biotechnology Information (NCBI) in the United States. Additionally, the amino acid sequence according to SEQ ID NO: 2 corresponds to the 90-th to 104-th positions in the amino acid sequence (SEQ ID NO: 37) obtained by removing the 1-st to the 20-th positions, which correspond to a signal peptide, from the amino acid sequence indicated by SEQ ID NO: 36.
SEQ ID NO: 2: LVLYNNADGDFAIVK

In one embodiment of the present disclosure, the full length of the (B) one or more polypeptides may be, for example, at least 12 residues, at least 13 residues, at least 14 residues, at least 15 residues, at least 16 residues, at least 17 residues, or at least 18 residues, and may be at most 21 residues, at most 20 residues, at most 19 residues, at most 18 residues, at most 17 residues, at most 16 residues, or at most 15 residues. Additionally, in one embodiment of the present disclosure, the full length of the (B) one or more polypeptides may be, for example, at least 12 residues and at most 21 residues, at least 13 residues and at most 21 residues, at least 14 residues and at most 21 residues, at least 15 residues and at most 21 residues, at least 15 residues and at most 19 residues, at least 15 residues and at most 17 residues, at least 15 residues and at most 16 residues, or 15 residues. In one preferable embodiment, the full length of the above-mentioned (B) one or more polypeptides may be at least 15 residues and at most 16 residues.

In one embodiment of the present disclosure, the above-mentioned (B) one or more polypeptides include an amino acid sequence having 90% or higher, 93% or higher, 95% or higher, 98% or higher, or 100% sequence identity with the amino acid sequence according to SEQ ID NO: 2. In one embodiment of the present disclosure, the above-mentioned (B) one or more polypeptides include the amino acid sequence according to SEQ ID NO: 2 or an amino acid sequence having a mutation at one residue, at two residues, or at three residues in said amino acid sequence. In one preferable embodiment, the above-mentioned (B) one or more polypeptides include an amino acid sequence having 93% or higher sequence identity with the amino acid sequence according to SEQ ID NO: 2.

In one embodiment of the present disclosure, the above-mentioned (B) one or more polypeptides may include an amino acid sequence having 90% or higher sequence identity with at least one amino acid sequence selected from the group consisting of the 87-th to 107-th positions, the 88-th to 107-th positions, the 89-th to 107-th positions, the 90-th to 107-th positions, the 87-th to 106-th positions, the 88-th to 106-th positions, the 89-th to 106-th positions, the 90-th to 106-th positions, the 87-th to 105-th positions, the 88-th to 105-th positions, the 89-th to 105-th positions, the 90-th to 105-th positions, the 87-th to 104-th positions, the 88-th to 104-th positions, the 89-th to 104-th positions, and the 90-th to 104-th positions in the amino acid sequence indicated by SEQ ID NO: 37, may comprise an amino acid sequence having 90% or higher sequence identity with said at least one amino acid sequence, or may comprise said at least one amino acid sequence.

In one embodiment of the present disclosure, the above-mentioned (B) one or more polypeptides may be (B') one or more polypeptides having 90% or higher sequence identity with an amino acid sequence selected from the group consisting of amino acid sequences according to SEQ ID NO: 16, or may be a polypeptide comprising the amino acid sequence according to SEQ ID NO: 16. The amino acid sequence indicated by SEQ ID NO: 16 corresponds to the 90-th to 104-th positions in the amino acid sequence indicated by SEQ ID NO: 37.
SEQ ID NO: 16: LVLYNNADGDFAIVK

The present inventors discovered that the above-mentioned (B) one or more polypeptides bind, among human HLAs, to HLA-DRB3*01:01. Therefore, the agent according to one embodiment of the present disclosure may be an agent for use in a subject in whom the HLA-DRB3 genotype is 01:01, wherein the above-mentioned at least one polypeptide includes the above-mentioned (B) one or more polypeptides.

The amino acid sequence according to SEQ ID NO: 4 corresponds to the 334-th to 343-rd positions in the amino acid sequence (SEQ ID NO: 36) of the α1 subunit of human acetylcholine receptor, having RefSeq accession no. NP_000070.1 at the National Center for Biotechnology Information (NCBI) in the United States. Additionally, the amino acid sequence according to SEQ ID NO: 4 corresponds to the 314-th to 323-rd positions in the amino acid sequence (SEQ ID NO: 37) obtained by removing the 1-st to the 20-th positions, which correspond to a signal peptide, from the amino acid sequence indicated by SEQ ID NO: 36.
SEQ ID NO: 4: KVFIDTIPNI

In one embodiment of the present disclosure, the full length of the (D) one or more polypeptides may be, for example, at least 7 residues, at least 8 residues, at least 9 residues, at least 10 residues, at least 11 residues, at least 12 residues, at least 13 residues, or at least 14 residues, and may be at most 17 residues, at most 16 residues, at most 15 residues, at most 14 residues, at most 13 residues, at most 12 residues, or at most 11 residues. Additionally, in one embodiment of the present disclosure, the full length of the above-mentioned (D) one or more polypeptides may be, for example, at least 7 residues and at most 17 residues, at least 8 residues and at most 17 residues, at least 9 residues and at most 17 residues, at least 10 residues and at most 17 residues, at least 11 residues and at most 16 residues, at least 11 residues and at most 14 residues, at least 11 residues and at most 13 residues, at least 11 residues and at most 12 residues, or 11 residues. In one preferable embodiment, the full length of the above-mentioned (D) one or more polypeptides may be at least 11 residues and at most 16 residues.

In one embodiment of the present disclosure, the above-mentioned (D) one or more polypeptides include an amino acid sequence having 90% or higher, 95% or higher, 98% or higher, or 100% sequence identity with the amino acid sequence according to SEQ ID NO: 4. In one embodiment of the present disclosure, the above-mentioned (D) one or more polypeptides include the amino acid sequence according to SEQ ID NO: 4 or an amino acid sequence having a mutation at one residue, at two residues, or at three residues in said amino acid sequence.

In one embodiment of the present disclosure, the above-mentioned (D) one or more polypeptides may include an amino acid sequence having 90% or higher sequence identity with at least one amino acid sequence selected from the group consisting of the 311-th to 327-th positions, the 312-th to 327-th positions, the 313-th to 327-th positions, the 314-th to 327-th positions, the 311-th to 326-th positions, the 312-th to 326-th positions, the 313-th to 326-th positions, the 314-th to 326-th positions, the 311-th to 325-th positions, the 312-th to 325-th positions, the 313-th to 325-th positions, the 314-th to 325-th positions, the 311-th to 324-th positions, the 312-th to 324-th positions, the 313-th to 324-th positions, and the 314-th to 324-th positions in the amino acid sequence indicated by SEQ ID NO: 37, may comprise an amino acid sequence having 90% or higher sequence identity with said at least one amino acid sequence, or may comprise said at least one amino acid sequence. For example, the 311-th to 327-th positions in the amino acid sequence indicated by SEQ ID NO: 37 correspond to the amino acid sequence indicated by SEQ ID NO: 17. In one embodiment of the present invention, the above-mentioned (D) one or more polypeptides may include an amino acid sequence having 90% or higher sequence identity with the amino acid sequence indicated by SEQ ID NO: 17, may comprise an amino acid sequence having 90% or higher sequence identity with the amino acid sequence indicated by SEQ ID NO: 17, or may comprise said amino acid sequence.
SEQ ID NO: 17: WVRKVFIDTIPNIMFFS

In one embodiment of the present disclosure, the above-mentioned (D) one or more polypeptides may be (D') one or more polypeptides having 90% or higher sequence identity with an amino acid sequence selected from the group consisting of amino acid sequences according to SEQ ID NOs: 22-32, or may be one or more polypeptides comprising an amino acid sequence selected from the group consisting of amino acid sequences according to SEQ ID NOs: 22-32. The amino acid sequence indicated by SEQ ID NO: 22 corresponds to the 311-th to 323-rd positions in the amino acid sequence indicated by SEQ ID NO: 37. The amino acid sequence indicated by SEQ ID NO: 23 corresponds to the 311-th to 324-th positions in the amino acid sequence indicated by SEQ ID NO: 37. The amino acid sequence indicated by SEQ ID NO: 24 corresponds to the 312-th to 324-th positions in the amino acid sequence indicated by SEQ ID NO: 37. The amino acid sequence indicated by SEQ ID NO: 25 corresponds to the 312-th to 325-th positions in the amino acid sequence indicated by SEQ ID NO: 37. The amino acid sequence indicated by SEQ ID NO: 26 corresponds to the 312-th to 327-th positions in the amino acid sequence indicated by SEQ ID NO: 37. The amino acid sequence indicated by SEQ ID NO: 27 corresponds to the 313-th to 324-th positions in the amino acid sequence indicated by SEQ ID NO: 37. The amino acid sequence indicated by SEQ ID NO: 28 corresponds to the 313-th to 325-th positions in the amino acid sequence indicated by SEQ ID NO: 37. The amino acid sequence indicated by SEQ ID NO: 29 corresponds to the 313-th to 326-th positions in the amino acid sequence indicated by SEQ ID NO: 37. The amino acid sequence indicated by SEQ ID NO: 30 corresponds to the 313-th to 327-th positions in the amino acid sequence indicated by SEQ ID NO: 37. The amino acid sequence indicated by SEQ ID NO: 31 corresponds to the 314-th to 324-th positions in the amino acid sequence indicated by SEQ ID NO: 37. The amino acid sequence indicated by SEQ ID NO: 32 corresponds to the 314-th to 326-th positions in the amino acid sequence indicated by SEQ ID NO: 37.
SEQ ID NO: 22: WVRKVFIDTIPNI
SEQ ID NO: 23: WVRKVFIDTIPNIM
SEQ ID NO: 24: VRKVFIDTIPNIM
SEQ ID NO: 25: VRKVFIDTIPNIMF
SEQ ID NO: 26: VRKVFIDTIPNIMFFS
SEQ ID NO: 27: RKVFIDTIPNIM
SEQ ID NO: 28: RKVFIDTIPNIMF
SEQ ID NO: 29: RKVFIDTIPNIMFF
SEQ ID NO: 30: RKVFIDTIPNIMFFS
SEQ ID NO: 31: KVFIDTIPNIM
SEQ ID NO: 32: KVFIDTIPNIMFF

The present inventors discovered that the above-mentioned (D) one or more polypeptides bind, among human HLAs, to HLA-DRB3*01:01 and HLA-DRB1*09:01. Therefore, the agent according to one embodiment of the present disclosure may be an agent for use in a subject in whom the HLA-DRB3 genotype is 01:01 and/or the HLA-DRB1 genotype is 09:01, wherein the above-mentioned at least one polypeptide includes the above-mentioned (D) one or more polypeptides,

The amino acid sequence according to SEQ ID NO: 5 corresponds to the 165-th to 177-th positions in the amino acid sequence (SEQ ID NO: 36) of the α1 subunit of human acetylcholine receptor, having RefSeq accession no. NP_000070.1 at the National Center for Biotechnology Information (NCBI) in the United States. Additionally, the amino acid sequence according to SEQ ID NO: 5 corresponds to the 145-th to 157-th positions in the amino acid sequence (SEQ ID NO: 37) obtained by removing the 1-st to the 20-th positions, which correspond to a signal peptide, from the amino acid sequence indicated by SEQ ID NO: 36.
SEQ ID NO: 5: KLGTWTYDGSVVA

In one embodiment of the present disclosure, the full length of the (E) one or more polypeptides may be, for example, at least 10 residues, at least 11 residues, at least 12 residues, at least 13 residues, at least 14 residues, at least 15 residues, or at least 16 residues, and may be at most 19 residues, at most 18 residues, at most 17 residues, at most 16 residues, at most 15 residues, at most 14 residues, or at most 13 residues. Additionally, in one embodiment of the present disclosure, the full length of the above-mentioned (E) one or more polypeptides may be, for example, at least 10 residues and at most 19 residues, at least 11 residues and at most 19 residues, at least 12 residues and at most 19 residues, at least 13 residues and at most 19 residues, at least 13 residues and at most 17 residues, at least 13 residues and at most 15 residues, at least 13 residues and at most 14 residues, or 13 residues. In one preferable embodiment, the full length of the above-mentioned (E) one or more polypeptides may be at least 13 residues and at most 17 residues.

In one embodiment of the present disclosure, the above-mentioned (E) one or more polypeptides include an amino acid sequence having 90% or higher, 92% or higher, 95% or higher, 98% or higher, or 100% sequence identity with the amino acid sequence according to SEQ ID NO: 5. In one embodiment of the present disclosure, the above-mentioned (E) one or more polypeptides include the amino acid sequence according to SEQ ID NO: 5 or an amino acid sequence having a mutation at one residue, at two residues, or at three residues in said amino acid sequence. In one preferable embodiment, the above-mentioned (E) one or more polypeptides include amino acid sequences having 92% or higher sequence identity with the amino acid sequence according to SEQ ID NO: 5.

In one embodiment of the present disclosure, the above-mentioned (E) one or more polypeptides may include an amino acid sequence having 90% or higher sequence identity with at least one amino acid sequence selected from the group consisting of the 142-nd to 161-st positions, the 143-rd to 161-st positions, the 144-th to 161-st positions, the 145-th to 161-st positions, the 142-nd to 160-th positions, the 143-rd to 160-th positions, the 144-th to 160-th positions, the 145-th to 160-th positions, the 142-nd to 159-th positions, the 143-rd to 159-th positions, the 144-th to 159-th positions, the 145-th to 159-th positions, the 142-nd to 158-th positions, the 143-rd to 158-th positions, the 144-th to 158-th positions, the 145-th to 158-th positions, the 142-nd to 157-th positions, the 143-rd to 157-th positions, the 144-th to 157-th positions, and the 145-th to 157-th positions in the amino acid sequence indicated by SEQ ID NO: 37, may comprise an amino acid sequence having 90% or higher sequence identity with said at least one amino acid sequence, or may comprise said at least one amino acid sequence.

In one embodiment of the present disclosure, the above-mentioned (E) one or more polypeptides may be (E') one or more polypeptides having 90% or higher sequence identity with an amino acid sequence selected from the group consisting of amino acid sequences according to SEQ ID NOs: 33-35, or may be one or more polypeptides comprising an amino acid sequence selected from the group consisting of amino acid sequences according to SEQ ID NOs: 33-35. The amino acid sequence indicated by SEQ ID NO: 33 corresponds to the 145-th to 157-th positions in the amino acid sequence indicated by SEQ ID NO: 37. The amino acid sequence indicated by SEQ ID NO: 34 corresponds to the 145-th to 160-th positions in the amino acid sequence indicated by SEQ ID NO: 37. The amino acid sequence indicated by SEQ ID NO: 35 corresponds to the 145-th to 161-st positions in the amino acid sequence indicated by SEQ ID NO: 37.
SEQ ID NO: 33: KLGTWTYDGSVVA
SEQ ID NO: 34: KLGTWTYDGSVVAINP
SEQ ID NO: 35: KLGTWTYDGSVVAINPE

The present inventors discovered that the above-mentioned (E) one or more polypeptides bind, among human HLAs, to HLA-DRB3*01:01 and HLA-DRB1*03:01. Therefore, the agent according to one embodiment of the present disclosure may be an agent for use in a subject in whom the HLA-DRB3 genotype is 01:01 and/or the HLA-DRB1 genotype is 03:01, wherein the above-mentioned at least one polypeptide includes the above-mentioned (E) one or more polypeptides.

The above-mentioned at least one polypeptide selected from the group consisting of (A), (B), (D), and (E) binds to HLA-DR. Therefore, though without wishing to be constrained to any theory, it appears that, by bringing the above-mentioned at least one polypeptide selected from the group consisting of (A), (B), (D), and (E) into contact with and causing phagocytosis in antigen-presenting cells having the function of accelerating the activation (differentiation) of regulatory T cells such as those described in Non-Patent Document 1, the above-mentioned at least one polypeptide is presented to naive CD4⁺ T cells in a state of being bound to HLA-DR, thereby inducing activation (differentiation) of the regulatory T cells.

The agent according to one embodiment may further contain one or more HLA-DRs in addition to the above-mentioned at least one polypeptide selected from the group consisting of (A), (B), (D), and (E). Said HLA-DRs preferably can bind the above-mentioned at least one polypeptide selected from the group consisting of (A), (B), (D), and (E), and a genotype among said HLA-DRs is preferably the same as the genotype of the HLA-DR in the subject to whom the agent is administered. The agent according to one embodiment may further contain an additive that is conventionally used in the technical field of drug preparation in addition to the above-mentioned at least one polypeptide selected from the group consisting of (A), (B), (D), and (E), and said additive may, for example, be at least one additive selected form the group consisting of excipients, buffering agents, stabilizers, antioxidants, binders, disintegrants, fillers, emulsifiers, and flow additives/adjusters.

A component contained in the agent according to one embodiment may be encapsulated in a drug carrier for the purpose of delivery into a cell. Such drug carriers include, for example, metal nanoparticles (for example, gold nanoparticles), liposomes, capsids, and the like.

The dosage form according to one embodiment is not particularly limited, and may, for example, be a liquid, a tablet, or the like. However, when the agent according to one embodiment contains a component encapsulated in a drug carrier for the purpose of delivery into a cell, the dosage form should preferably be a liquid.

The route of administration of the agent according to one embodiment may be, for example, intramedullary administration, intravenous injection, subcutaneous injection, intramuscular injection, oral administration, intrathecal injection, or the like. As one example of a specific dose, for example, in the case of administration to an adult human male (body weight 60 kg), the daily dose of the agent may be 0.001 µg to 1000 mg per day per person in terms of the amount of the active ingredient.

The content of the above-mentioned at least one polypeptide selected from the group consisting of (A), (B), (D), and (E) in the agent according to one embodiment is not particularly limited, and for example, the total content thereof may be 0.001 to 100 mass% with respect to the total amount of the agent.

The subject of administration of the agent according to one embodiment may be, for example, a human, and may be a human who is suffering from, who may be suffering from, or who has genetic factors causing susceptibility to an autoimmune disease. The above-mentioned autoimmune disease may be, for example, an autoimmune disease caused by overproduction of antibodies to human AChRα1 or peptide fragments thereof, is preferably myasthenia gravis, and is more preferably early-onset myasthenia gravis. Myasthenia gravis can be largely divided into early-onset myasthenia gravis, which occurs in subjects aged from 10 to 49 years, and late-onset myasthenia gravis, which occurs in subjects aged 50 years or older. The genotypes of HLA that can be risk factors partially differ between these two types.

The above-mentioned at least one polypeptide selected from the group consisting of (A), (B), (D), and (E) was discovered by means of a NAPA method using dendritic cells prepared from classical monocytes from humans, and is highly likely to be actually present in the body of a patient suffering from an autoimmune disease. Therefore, it has the potential to be more suitable for application to the treatment or diagnosis of autoimmune diseases than polypeptides with sequences corresponding to epitopes that have been discovered previously. In this case, the fact that the above-mentioned at least one polypeptide selected from the group consisting of (A), (B), (D), and (E) is more suitable for application to the treatment or diagnosis of autoimmune diseases than polypeptides with sequences corresponding to epitopes that have been discovered previously (other polypeptides) means that, for example, the above-mentioned at least one polypeptide can be evaluated based on its higher T cell activation activity when brought into contact with T cells, than the above-mentioned other polypeptides. Such a T cell activation activity can be evaluated by means of, for example, a T cell activation test. A T cell activation test is a test for checking, by ELISA for IFN-γ or the like, whether or not T cells present among PBMCs are activated by exposure to polypeptides. When a polypeptide with a sequence corresponding to a discovered epitope is added to human PBMCs, the polypeptide is presented as an epitope by antigen-presenting cells among the PBMCs. T cells having TCRs that can recognize HLA-binding polypeptides receive signals and thereby release activation cytokines such as IFN-γ. These released cytokines are measured to assess the activation. Such assays can be performed by using commercially available kits. For example, they can be performed by using BD OptEIA^{®} Human IFN-γ ELISA set (BD Biosciences, Cat. No. 555142).

<Activity inhibitory agent or composition for inhibiting activity of effector T cells>
A second aspect of the present disclosure relates to an activity inhibitory agent for effector T cells, containing the above-mentioned at least one polypeptide selected from the group consisting of (A), (B), (D), and (E). The second aspect of the present disclosure, in one embodiment, may also be a composition for inhibiting activity of effector T cells, containing the above-mentioned at least one polypeptide. As the activity inhibitory agent for effector T cells according to the second aspect of the present disclosure, agents similar to those explained for the activating agent for regulatory T cells in the first aspect of the present disclosure can be used. The explanations and definitions provided in the section regarding the activating agent for regulatory T cells according to the first aspect of the present disclosure also apply to the activity inhibitory agent for effector T cells according to the second aspect of the present disclosure.

Without wishing to be constrained to any theory, it appears that the activity inhibitory agent for effector T cells containing the above-mentioned at least one polypeptide selected from the group consisting of (A), (B), (D), and (E) accelerates the activation (differentiation) of regulatory T cells through the action of antigen-presenting cells such as those described in Non-Patent Document 1, as explained for the first aspect of the present disclosure, and is thereby able to inhibit the activity of effector T cells.

### <Activation inhibitory agent or composition for inhibiting activation of B cells>

A third aspect of the present disclosure relates to an activation inhibitory agent for B cells, containing the above-mentioned at least one polypeptide selected from the group consisting of (A), (B), (D), and (E). The third aspect of the present disclosure, in one embodiment, may also be a composition for inhibiting activation of B cells, containing the above-mentioned at least one polypeptide. As the activation inhibitory agent for B cells according to the third aspect of the present disclosure, agents similar to those explained for the activating agent for regulatory T cells in the first aspect of the present disclosure can be used. The explanations and definitions provided in the section regarding the activating agent for regulatory T cells according to the first aspect of the present disclosure also apply to the activation inhibitory agent for B cells according to the third aspect of the present disclosure.

Without wishing to be constrained to any theory, it appears that the activation inhibitory agent for B cells containing the above-mentioned at least one polypeptide selected from the group consisting of (A), (B), (D), and (E) can inhibit the activity of effector T cells through the action of antigen-presenting cells such as those described in Non-Patent Document 1, as explained for the second aspect of the present disclosure, and is thereby able to inhibit the activation of B cells.

### <Suppressive agent or composition for suppression of humoral immunity>

A fourth aspect of the present disclosure relates to a suppressive agent (for example, an antibody production suppressive agent) for humoral immunity, containing the above-mentioned at least one polypeptide selected from the group consisting of (A), (B), (D), and (E). The fourth aspect of the present disclosure, in one embodiment, may also be a composition for suppressing humoral immunity, containing the above-mentioned at least one polypeptide. As the suppressive agent for humoral immunity according to the fourth aspect of the present disclosure, agents similar to those explained for the activating agent for regulatory T cells in the first aspect of the present disclosure can be used. The explanations and definitions provided in the section regarding the activating agent for regulatory T cells according to the first aspect of the present disclosure also apply to the suppressive agent for humoral immunity according to the fourth aspect of the present disclosure.

Without wishing to be constrained to any theory, it appears that the suppressive agent for humoral immunity containing the above-mentioned at least one polypeptide selected from the group consisting of (A), (B), (D), and (E) can inhibit the activation of B cells through the action of antigen-presenting cells such as those described in Non-Patent Document 1, as explained for the third aspect of the present disclosure, and is thereby able to suppress humoral immunity.

### <Therapeutic agent, preventive agent, composition for treatment, or composition for prevention of autoimmune disease>

A fifth aspect of the present disclosure relates to a therapeutic agent or a preventive agent for an autoimmune disease, containing the above-mentioned at least one polypeptide selected from the group consisting of (A), (B), (D), and (E). The fifth aspect of the present disclosure, in one embodiment, may also be a composition for treatment of or a composition for prevention of an autoimmune disease, containing the above-mentioned at least one polypeptide. The fifth aspect of the present disclosure, in one embodiment, may also be a treatment method or a prevention method for an autoimmune disease, wherein the method includes administering, to a subject in need thereof, the above-mentioned at least one polypeptide. The fifth aspect of the present disclosure, in one embodiment, may also be the above-mentioned at least one polypeptide, for use in the treatment or prevention of an autoimmune disease. The fifth aspect of the present disclosure, in one embodiment, may also be use of the above-mentioned at least one polypeptide in the production of a therapeutic agent, a preventive agent, a composition for treatment, or a composition for prevention of an autoimmune disease. The fifth aspect of the present disclosure, in one embodiment, may also be the above-mentioned at least one polypeptide for use in the production of a therapeutic agent, a preventive agent, a composition for treatment, or a composition for prevention of an autoimmune disease. As the therapeutic agent or the preventive agent for an autoimmune disease according to the fifth aspect of the present disclosure, agents similar to those explained for the activating agent for regulatory T cells in the first aspect of the present disclosure can be used. The explanations and definitions provided in the section regarding the activating agent for regulatory T cells according to the first aspect of the present disclosure also apply to the therapeutic agent or the preventive agent for autoimmune disease according to the fifth aspect of the present disclosure.

The autoimmune disease according to the fifth aspect of the present disclosure may be, for example, an autoimmune disease caused by overproduction of antibodies to human AchRα1 or peptide fragments thereof, is preferably myasthenia gravis, and is more preferably early-onset myasthenia gravis. The above-mentioned at least one polypeptide selected from the group consisting of (A), (B), (D), and (E) is a peptide fragment including an amino acid sequence corresponding to an epitope of human AchRα1, which is known to be recognized as an autoantigen in myasthenia gravis and early-onset myasthenia gravis. Therefore, the agent according to one embodiment of the fifth aspect of the present disclosure can favorably treat and prevent myasthenia gravis and early-onset myasthenia gravis.

The therapeutic agent or preventive agent according to one embodiment of the fifth aspect of the present disclosure may, for example, be for administration to a human, and may be for administration to a human who is suffering from or who may be suffering from, or who has genetic factors causing susceptibility to an autoimmune disease. The above-mentioned autoimmune disease may be, for example, an autoimmune disease caused by overproduction of antibodies to human AchRα1 or peptide fragments thereof, is preferably myasthenia gravis, and is more preferably early-onset myasthenia gravis. Additionally, in the case in which the agent according to one embodiment of the fifth aspect of the present disclosure is a therapeutic agent, the subject of administration thereof may, for example, be a human who is suffering from or who may be suffering from an autoimmune disease. Additionally, in the case in which the agent according to one embodiment of the fifth aspect of the present disclosure is a preventive agent, the subject of administration thereof may, for example, be a human who may be suffering from or who has genetic factors causing susceptibility to an autoimmune disease.

Without wishing to be constrained to any theory, it appears that the therapeutic agent or the preventive agent for an autoimmune disease containing the above-mentioned at least one polypeptide selected from the group consisting of (A), (B), (D), and (E) can suppress humoral immunity through the action of antigen-presenting cells such as those described in Non-Patent Document 1, as explained for the fourth aspect of the present disclosure, and is thereby able to treat and prevent autoimmune diseases. For example, it appears to suppress the decomposition of AchRα1 by suppressing the production of anti-AchRα1, as a result of which myasthenia gravis, such as early-onset myasthenia gravis, can be treated and prevented.

### <Diagnosis aiding method, method for acquiring data for diagnosis, and diagnosis method for autoimmune disease>

A sixth aspect of the present disclosure relates to a method for aiding diagnosis of an autoimmune disease, wherein the method includes detecting T cells having antigen specificity to the above-mentioned at least one polypeptide selected from the group consisting of (A), (B), (D), and (E). The sixth aspect of the present disclosure, in one embodiment, may be a method for acquiring data for diagnosis of an autoimmune disease, wherein the method includes detecting T cells having antigen specificity to the above-mentioned at least one polypeptide selected from the group consisting of (A), (B), (D), and (E). The sixth aspect of the present disclosure, in one embodiment, may be a diagnosis method for an autoimmune disease, wherein the method includes detecting T cells having antigen specificity to the above-mentioned at least one polypeptide selected from the group consisting of (A), (B), (D), and (E). In these cases, the autoimmune disease may be, for example, an autoimmune disease caused by overproduction of antibodies to human AchRα1 or peptide fragments thereof, is preferably myasthenia gravis, and is more preferably early-onset myasthenia gravis. In these cases, as the above-mentioned at least one polypeptide selected from the group consisting of (A), (B), (D), and (E), a polypeptide similar to those explained for the activating agent for regulatory T cells according to the first aspect of the present disclosure can be used. The explanations and definitions of the above-mentioned at least one polypeptide provided in the section regarding the activating agent for regulatory T cells according to the first aspect of the present disclosure also apply to the above-mentioned at least one polypeptide in the method according to the sixth aspect of the present disclosure.

In a subject who is suffering from an autoimmune disease (for example, an autoimmune disease caused by overproduction of antibodies to human AchRα1 or peptide fragments thereof, preferably myasthenia gravis, and more preferably early-onset myasthenia gravis), T cells having antigen specificity to the above-mentioned at least one polypeptide selected from the group consisting of (A), (B), (D), and (E), i.e., T cells expressing antibodies recognizing the above-mentioned at least one polypeptide selected from the group consisting of (A), (B), (D), and (E) on the cell surfaces thereof, are more easily produced. Thus, by using the presence or the concentration as an indicator of the amount of T cells having antigen specificity to the above-mentioned at least one polypeptide selected from the group consisting of (A), (B), (D), and (E), it is possible to aid diagnosis, to acquire data for diagnosis, or to perform a diagnosis regarding whether or not a person is suffering from an autoimmune disease.

The subject for whom the method according to one embodiment of the sixth aspect of the present disclosure is to be used may, for example, be a human, and may be a human who may be suffering from, or who has genetic factors causing susceptibility to an autoimmune disease. The autoimmune disease may be, for example, an autoimmune disease caused by overproduction of antibodies to human AchRα1 or peptide fragments thereof, is preferably myasthenia gravis, and is more preferably early-onset myasthenia gravis. Additionally, the subject for whom the method according to one embodiment of the sixth aspect of the present disclosure is to be used may be a human not having the possibility of suffering from, or genetic factors causing susceptibility to, such an autoimmune disease, and in that case, the method according to one embodiment may be a method for use in comprehensive discovery of the possibility of suffering from a disease (a so-called screening test).

In one embodiment of the sixth aspect of the present disclosure, it is possible to aid in diagnosis, to acquire data for diagnosis, or to perform a diagnosis that there is a possibility that a subject is suffering from an autoimmune disease (for example, an autoimmune disease caused by overproduction of antibodies to human AchRα1 or peptide fragments thereof, preferably myasthenia gravis, and more preferably early-onset myasthenia gravis) when T cells having antigen specificity to the above-mentioned at least one polypeptide selected from the group consisting of (A), (B), (D), and (E) are detected in a sample taken from the subject. Additionally, in one embodiment of the sixth aspect of the present disclosure, it is possible to aid in diagnosis, to acquire data for diagnosis, or to perform a diagnosis that there is a high probability that a subject is suffering from an autoimmune disease (for example, an autoimmune disease caused by overproduction of antibodies to human AchRα1 or peptide fragments thereof, preferably myasthenia gravis, and more preferably early-onset myasthenia gravis) when the amount of T cells having antigen specificity to the above-mentioned at least one polypeptide selected from the group consisting of (A), (B), (D), and (E) in a sample taken from the subject is greater than that in a sample taken from a control (for example, a healthy donor) not suffering from said autoimmune disease.

The sample in which the amount of T cells having antigen specificity to the above-mentioned at least one polypeptide selected from the group consisting of (A), (B), (D), and (E) is measured is not limited as long as it is a sample which can be acquired from a subject and with which it is possible to aid in diagnosis, to acquire data for diagnosis, or to perform a diagnosis of an autoimmune disease based on the amount of T cells having antigen specificity to the above-mentioned at least one polypeptide selected from the group consisting of (A), (B), (D), and (E). For example, it may be bone marrow fluid, cerebrospinal fluid, lymph fluid, blood, blood plasma, blood serum, or saliva. In one preferable embodiment, it may be bone marrow fluid, and said bone marrow fluid may be taken from the ilium or the sternum.

In the sixth aspect of the present disclosure, the "amount" of the T cells having antigen specificity to the above-mentioned at least one polypeptide selected from the group consisting of (A), (B), (D), and (E) is not limited to the concentration of said T cells, and may be the intensity of a signal that can be used as an indicator of the amount of said T cells. For example, in the case in which said T cells are labeled with a polypeptide, the above-mentioned amount may be the intensity of a signal by which the amount of said labeled polypeptide can be evaluated. For example, in the case in which the polypeptide is fluorescently labeled, it may be the fluorescent intensity detected from T cells. Additionally, in the case in which said T cells are labeled with a polypeptide-human leukocyte antigen complex, the above-mentioned amount may be the intensity of a signal by which the amount of said labeled complex can be evaluated, and for example, if the human leukocyte antigen complex is fluorescently labeled, it may be the fluorescent intensity detected from T cells. In these cases, the fluorescent intensity may be the fluorescent intensity measured, for example, by a microwell plate reader, a flow cytometer, or the like.

The detection method when detecting the T cells having antigen specificity to the above-mentioned at least one polypeptide selected from the group consisting of (A), (B), (D), and (E) is not particularly limited as long as the method can detect said T cells. For example, it can be performed by labeling the above-mentioned T cells with fluorescently labeled polypeptides or with complexes of polypeptide and fluorescently labeled human leukocyte antigens, then measuring the fluorescent intensity of the above-mentioned fluorescent dye in cells contained in a sample.

In one preferable embodiment, the T cells having antigen specificity to the above-mentioned at least one polypeptide selected from the group consisting of (A), (B), (D), and (E) may be detected by labeling said T cells with a multimer (for example, a tetramer) of a fluorescently labeled complex of the polypeptide with the human leukocyte antigen (polypeptide-human leukocyte antigen complex), then measuring the fluorescent intensity of the above-mentioned fluorescent dye in cells contained in a sample. In the case in which the above-mentioned T cells are labeled with a multimer of a polypeptide-human leukocyte antigen complex, said complex and the above-mentioned T cells can form stable bonds. Therefore, more accurate detection is made possible by using a washing operation or the like to remove said complexes that have been non-specifically bound. Such polypeptide-human leukocyte antigen complexes can be prepared by using, for example, the above-mentioned at least one polypeptide selected from the group consisting of (A), (B), (D), and (E) and a commercially available kit (for example, the Quick Switch^{®} series, MEDICAL & BIOLOGICAL LABORATORIES CO., LTD. (MBL)) that can be used to prepare a multimer of a polypeptide-human leukocyte antigen complex as explained above.

### <Kit for diagnosing autoimmune disease>

A seventh aspect of the present disclosure is a kit for diagnosing an autoimmune disease, containing the above-mentioned at least one polypeptide selected from the group consisting of (A), (B), (D), and (E), and a human leukocyte antigen that binds to the above-mentioned at least one polypeptide. The kit according to the seventh aspect of the present disclosure, in one embodiment, may further contain a labeling dye that binds to the human leukocyte antigen. The kit according to the seventh aspect of the present disclosure, in one embodiment, may contain the above-mentioned human leukocyte antigen and the above-mentioned labeling dye as a complex, may contain the above-mentioned human leukocyte antigen and the above-mentioned labeling dye as a complex, may contain the polypeptide, the above-mentioned human leukocyte antigen, and the above-mentioned labeling dye as a complex, may contain a multimer of the above-mentioned human leukocyte antigen and a labeling dye as a complex, or may contain a multimer of the polypeptide-human leukocyte antigen complex and the labeling dye as a complex. That is, the seventh aspect of the present disclosure, in one embodiment, may be a diagnostic agent for an autoimmune disease containing a complex comprising a polypeptide, a human leukocyte antigen, and a labeling dye. In these cases, the labeling dye may be a fluorescent dye. In these cases, the autoimmune disease may, for example, be an autoimmune disease caused by overproduction of antibodies to human AchRα1 or peptide fragments thereof, is preferably myasthenia gravis, and is more preferably early-onset myasthenia gravis.

The kit according to the seventh aspect of the present disclosure can be used to diagnose an autoimmune disease in accordance with the method according to one embodiment of the sixth aspect of the present disclosure. The kit according to the seventh aspect of the present disclosure, in one embodiment, may further contain an attached document, said attached document may be electronic, and said attached document may describe a protocol corresponding to the method according to one embodiment of the sixth aspect of the present disclosure.

### <Modification examples>

An eighth aspect of the present disclosure relates to an agent and a kit wherein the agent, the method, and the kit explained above contain instead of the at least one polypeptide selected from the group consisting of (A), (B), (D), and (E), a molecule including, as a partial structure, the at least one polypeptide selected from the group consisting of (A), (B), (D), and (E) (hereinafter also referred to as a "molecule according to the eighth aspect"). Such an agent and kit can be implemented similarly to what has been explained above regarding the agent according to the first aspect to the fifth aspect and the kit according to the seventh aspect, aside from the feature of containing the molecule according to the eighth aspect instead of the above-mentioned at least one polypeptide. The molecule according to the eighth aspect may be a non-natural molecule, and for example, may be an artificial molecule.

The molecule according to the eighth aspect may be, for example, a molecule (tandem molecule) in which at least two polypeptides selected from the group consisting of (A), (B), (D), and (E) are directly or indirectly linked via peptide bonds. The tandem molecule may be linear or cyclic. The tandem molecule may be a molecule (tandem molecule) in which, for example, two, three, four, or five or more polypeptides selected from the group consisting of (A), (B), (D), and (E) are directly or indirectly linked via peptide bonds. As the tandem molecule in which at least two polypeptides are directly or indirectly linked via peptide bonds, there are, for example, a polypeptide comprising an amino acid sequence in which the amino acid sequence indicated by SEQ ID NO: 17 occurs twice consecutively, as indicated by SEQ ID NO: 18, a polypeptide comprising an amino acid sequence in which the amino acid sequence indicated by SEQ ID NO: 17 and the amino acid sequence indicated by SEQ ID NO: 3 occur consecutively, as indicated by SEQ ID NO: 19, a cyclic polypeptide in which a peptide bond is formed at both ends thereof, or the like. The tandem molecule in which at least two polypeptides are indirectly linked via peptide bonds may be, for example, a molecule in which the at least two polypeptides are bound by a peptide linker. As said peptide linker, for example, a peptide linker disclosed in WO 2019/233842 A1 may be used. Specific examples include GGGS (SEQ ID NO: 38), GGGGS (SEQ ID NO: 39), the autolytic peptide T2A (EGRGSLLTCGDVEENPGP (SEQ ID NO: 40) and GSGEGRGSLLTCGDVEENPGP (SEQ ID NO: 41)), or the like. The tandem molecule in which at least two polypeptides are indirectly bound by a peptide bond may be, for example, a polypeptide comprising an amino acid sequence in which GGGS is inserted between two amino acid sequences indicated by SEQ ID NO: 17, as indicated by SEQ ID NO: 20, a polypeptide comprising an amino acid sequence in which GGGGS is inserted respectively between the amino acid sequence indicated by SEQ ID NO: 17 and the amino acid sequence indicated by SEQ ID NO: 3, and between the amino acid sequence indicated by SEQ ID NO: 3 and the amino acid sequence indicated by SEQ ID NO: 17, as indicated by SEQ ID NO: 21, or the like.
SEQ ID NO: 18: WVRKVFIDTIPNIMFFSWVRKVFIDTIPNIMFFS
SEQ ID NO: 19: WVRKVFIDTIPNIMFFSGDFAIVKFTKVLLQYTGHIT
SEQ ID NO: 20: WVRKVFIDTIPNIMFFSGGGSWVRKVFIDTIPNIMFFS

SEQ ID NO: 21: WVRKVFIDTIPNIMFFSGGGGSGDFAIVKFTKVLLQYTGHITGGG
GSWVRKVFIDTIPNIMFFS

The molecule according to the eighth aspect may be, for example, a molecule including, as partial structures, at least one polypeptide selected from the group consisting of (A), (B), (D), and (E), and a structure that is not a peptide. Such a molecule may, for example, be a molecule in which at least two polypeptides selected from the group consisting of (A), (B), (D), and (E) are bound by a bond other than a peptide bond, and such a molecule may further have a structure that is not a peptide at a peptide end that is not bound to a linker. Such a bond other than a peptide bond may be a covalent bond (for example, a carbon-carbon bond), or may be a bond due to a non-covalent binding interaction typified by the interaction between biotin and streptavidin.

### EXAMPLES

Hereinafter, the present disclosure will be explained in further detail by using examples. However, the present disclosure is not limited to the examples below.

### [Example 1: Identification of antigen peptide from human AChRα1 binding to HLA-DR]

### <Step 1: Collection of clinical samples>

The peripheral blood mononuclear cells (hereinafter referred to as PBMCs) used in the present example were frozen samples from healthy donors, purchased from VERITAS Corp. The genotypes of the HLAs in the purchased PBMCs are indicated in Table 1 below.

**[Table 1]**

| | A | | B | | C | | DRB1 | | DRB3/4/5 | | DQB1 | | DPB1 | |
|---|---|---|---|---|---|---|---|---|---|---|---|---|---|---|
| Donor1 | 02:01 | 02.01 | 08.01 | 08:01 | 07:01 | 07:01 | 03:01 | 03:01 | 3*01:01 | 3*01:01 | 02:01 | 02:01 | 04:01 | 14:01 |
| Donor2 | 01:01 | 02:01 | 08-01 | 40:01 | 03:04 | 07:01 | 01:01 | 13:01 | 3*01:01 | 3*01:01 | 05:04 | 06:03 | 03:01 | 04:01 |
| Donor3 | 01:01 | 01:01 | 08.01 | 57:01 | 06:02 | 07:01 | 03:01 | 07:01 | 3*01:01 | 4*01:03 | 02:01 | 03:03 | 01:01 | 04:01 |
| Donor4 | 26:01 | 32:01 | 08.01 | 51:01 | 07:02 | 07:02 | 03:01 | 04:02 | 3*02:02 | 4*01:01 | 02:01 | 03:02 | 02:01 | 04:01 |
| Donor5 | 11:01 | 29:02 | 44:03 | 56:01 | 01:02 | 16:01 | 11:01 | 13:01 | 3*01:01 | 3*02:02 | 03:01 | 06:03 | 16:01 | 23:01 |
| Donor6 | 01:01 | 68:02 | 14:02 | 15:01 | 03:03 | 08:02 | 13:01 | 13:03 | 3*01:01 | 3*02:02 | 03:01 | 06:03 | 02:01 | 04:01 |
| Donor7 | 02:01 | 30:02 | 07:02 | 18:01 | 05:01 | 07:02 | 03:01 | 07:01 | 3*02:02 | 4*01:01 | 02:01 | 02:01 | 03:01 | 11:01 |
| Donor8 | 01:01 | 01:01 | 08:01 | 08:01 | 07:01 | 07:01 | 03:01 | 03:01 | 3*01:01 | 3*01:01 | 02:01 | 02:01 | 01:01 | 02:01 |

### <Step 2: Isolation of CD14⁺CD16⁻ monocytes>

The frozen PBMCs were quickly thawed in a 37 °C water bath, and washed twice with an RPMI-1640 culture medium (RPMI-1640/10% FBS culture medium) containing 10% FBS. An EasySep Human Monocyte Isolation Kit (VERITAS, Cat. No. ST-19359) was used to isolate CD14⁺CD16⁻ monocytes by negative selection in accordance with the instructions attached to the kit. As a result thereof, classical monocytes were isolated from the PBMCs.

### <Step 3: Production of monocyte-derived dendritic cells and addition of human AChRα1>

Culturing of the isolated CD14⁺CD16⁻ monocytes was initiated with 1.5 × 10⁶ cells/mL per well in a 6-well dish in an RPMI-1640/10% FBS culture medium containing 50 ng/mL of GM-CSF and 50 ng/mL of IL-4, and the cells were cultured for 7 days in a 5% CO₂ incubator at 37 °C to produce monocyte-derived dendritic cells. Thereafter, human AChRα1 was added so as to be 100 µg/well, and the cells were further cultured for 24 hours. The human AChRα1 used in the present step was prepared by performing gene synthesis based on AChRα sequence information, inserting the synthesized gene in a transfer vector, then cotransfecting SF9 cells, which are insect cells, with the transfection vector and the expression vector, and producing and purifying the human AChRα1.

### <Step 4: Isolation of HLA-DR-binding peptide>

The cultured sample was treated in accordance with the protocol for Dynabeads Protein G (Invitrogen, Cat. No. 10004D). 5 µg of anti-HLA-DR antibody was bound to the beads. 0.1% TFA was added to the resulting solution containing HLA-DR to which the peptide was bound, and a reaction was allowed to proceed for 1 minute at room temperature, thereby freeing the peptide from the HLA-DR. A Microcon^{®} Centrifugal Filter Device (Merck, Cat. No. MRCPRT010) capable of separating molecules that are 10kD or less was attached to a dedicated 1.5 mL tube, the collected solution containing the peptide and the HLA-DR was added thereto, and the solution was centrifuged for 30 minutes at 14000×g, thereby separating the HLA-DR from the peptide. The solvent was removed from the peptide solution in the tube by means of a lyophilizer, and preserved as a lyophilized sample.

### <Step 5: LC-MS/MS analysis>

Purified water was added to the resulting lyophilized sample and agitated to form a measurement sample. This was subjected to LC-MS/MS analysis under the conditions indicated in Table 2 below.

**[Table 2]**

| | |
|---|---|
| LC | EASY-nLC 1200 (ThermoFisher Scientific) |
| Column | NTCC-360/75-3-125 (Nikkyo Technos) |
| Mobile phase A | 0.1% (v/v) Formic acid, water |
| Mobile phase B | 0.1% (v/v) Formic acid, acetonitrile |
| Flow rate | 300 nL/min |
| Injection amount | 5 µL |
| Gradient | Mobile phase A: B = 100:0 (0.00 min) → 60:40 (12.30 min) |
| | → 5:95 (15.00 min) → 5:95 (25.00 min) |
| MS | Q Exactive Plus (ThermoFisher Scientific) |
| Ionization method | NSI pos (Spray voltage: 2000V) |
| Capillary temperature | 250 °C |
| Full MS scan range | m/z 350-2000 |
| Top N MSMS | 10 |

### <Step 6: LC-MS/MS spectral data analysis>

Mascot Server ver. 2.8 (Matrix Science) was used for analysis of the spectral data obtained by the LC-MS/MS analysis, and the analysis was performed under the conditions indicated in Table 3 below. In sequence searches in NCBI prot and AChRα1, the search target was set to be all peptides satisfying the condition that the false discovery rate (FDR) p < 0.01, and the FDR was not calculated for searches of only the AChRα1 sequence. Peptides satisfying the condition that the false discovery rate (FDR) p < 0.01 were the search targets. Additionally, Skyline ver. 22.2 (MacCoss Lab.) was used for quantitative analysis. After the spectral data obtained by the LC-MS/MS analysis and the identification results obtained by Mascot Server were loaded, the peak area values in extracted ion chromatograms of the peptides were calculated.

**[Table 3] Mascot Server Analysis Conditions**

| Reference database | NCBI prot sequences only, or His-AChR *α* 1 sequences only |
|---|---|
| Taxonomy | Homo sapiens |
| Search engine | Mascot |
| Enzyme | None |
| Fixed modification | Gin > pyro-Gln (N-term Q) |
| Variable modification | Oxidation(M), Deamidated(NQ) |

### <Step 7: Identification of HLA alleles by sample comparison>

In order to identify HLA alleles in the LC-MS/MS analysis data obtained in step 6, comparisons were performed with respective donors. Peptides that were observed in common with samples having DRB1*03:01 were identified as DRB1*03:01-binding peptides, and peptides that were observed in common with samples having DRB3*01:01 were identified as DRB3*01:01-binding peptides.

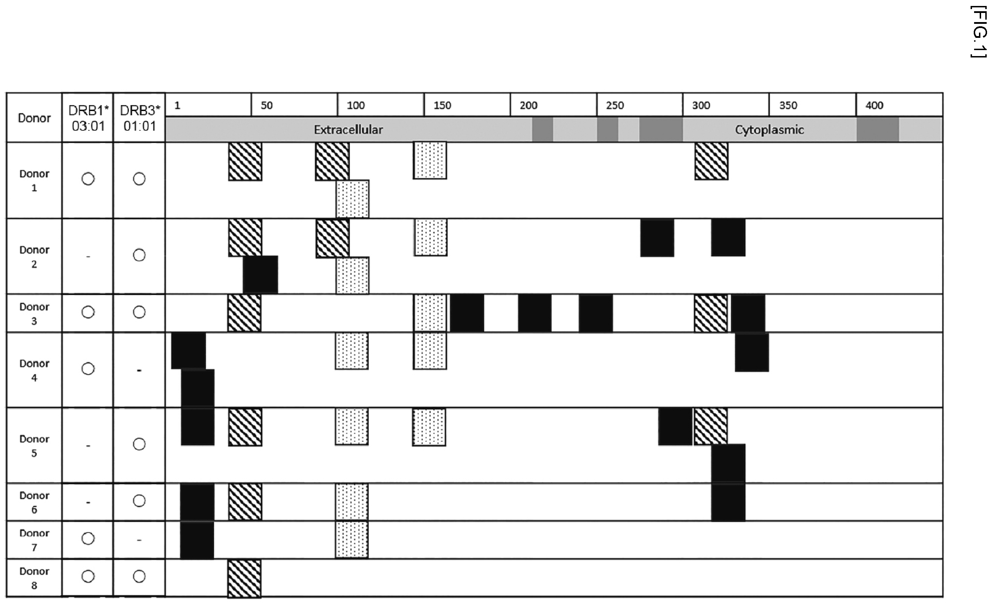

Results indicating HLA-DRB1*03:01-binding peptides identified in this way together with the corresponding positions in the amino acid sequence indicated by SEQ ID NO: 37 are shown in Table 4. Results indicating HLA-DRB3*01:01-binding peptides identified in this way together with the corresponding positions in the amino acid sequence indicated by SEQ ID NO: 37 are shown in Table 5. Results in which the HLA-binding peptides identified in this way are indicated as coordinates on human AChRα1 are shown in FIG. 1. In FIG. 1, identified DRB-binding peptide clusters are indicated as boxes, the clusters indicated by diagonal hatching being from DRB1*03:01, the clusters indicated by dot hatching being from DRB3*01:01, and the clusters indicated in solid black being from other HLAs. In FIG. 1, the light gray portions in the line below the amino acid numbers indicate the AChRα1 sequence, and the dark gray portions indicate transmembrane portions.

**[Table 4]**

| SEQ ID NO | Position in SEQ ID NO: 37 | Sequence |
|---|---|---|
| 6 | 98-111 | GDFAIVKFTKVLLQ |
| 7 | 98-113 | GDFAIVKFTKVLLQYT |
| 8 | 98-114 | GDFAIVKFTKVLLQYTG |
| 9 | 99-111 | DFAIVKFTKVLLQ |
| 10 | 99-113 | DFAIVKFTKVLLQYT |
| 11 | 99-114 | DFAIVKFTKVLLQYTG |
| 12 | 100-111 | FAIVKFTKVLLQ |
| 13 | 100-114 | FAIVKFTKVLLQYTG |
| 14 | 101-114 | AIVKFTKVLLQYTG |
| 15 | 101-117 | AIVKFTKVLLQYTGHIT |
| 33 | 145-157 | KLGTWTYDGSVVA |
| 34 | 145-160 | KLGTWTYDGSVVAINP |
| 35 | 145-161 | KLGTWTYDGSVVAINPE |

**[Table 5]**

| SEQ ID NO | Position in SEQ ID NO: 37 | Sequence |
|---|---|---|
| 16 | 90-104 | LVLYNNADGDFAIVK |
| 6 | 98-111 | GDFAIVKFTKVLLQ |
| 7 | 98-113 | GDFAIVKFTKVLLQYT |
| 8 | 98-114 | GDFAIVKFTKVLLQYTG |
| 9 | 99-111 | DFAIVKFTKVLLQ |
| 10 | 99-113 | DFA I VKFTKVLLQYT |
| 11 | 99-114 | DFAIVKFTKVLLQYTG |
| 12 | 100-111 | FAIVKFTKVLLQ |
| 13 | 100-114 | FAIVKFTKVLLQYTG |
| 14 | 101-114 | AIVKFTKVLLQYTG |
| 15 | 101-117 | AIVKFTKVLLQYTGHIT |
| 33 | 145-157 | KLGTWTYDGSYYA |
| 34 | 145-160 | KLGTWTYOGSVVAINP |
| 35 | 145-161 | KLGTWTYDGSVVAINPE |
| 22 | 311-323 | WVRKVFIDTIPNI |
| 23 | 311-324 | WVRKVEIDTIPNIM |
| 24 | 312-324 | VRKVFIDT IPNIM |
| 25 | 312-325 | VRKVFIDT IPNIMF |
| 26 | 312-327 | VRKVFIDTIPNIMFFS |
| 27 | 313-324 | RKVFIDTIPNIM |
| 28 | 313-325 | RKVFIDT IPNIMF |
| 29 | 313-326 | RKVFIDTIPNIMFF |
| 30 | 313-327 | RKVFIDTIPNIMFFS |
| 31 | 314-324 | KVFIDTIPNIM |
| 32 | 314-326 | KVFIDTIPNIMFF |

From these results, the peptides indicated by SEQ ID NOs: 6-15 were observed in common in the samples from subjects in whom the HLA-DRB3 genotype was 01:01 and the samples from subjects in whom the HLA-DRB1 genotype was 03:01, and they shared the amino acid sequence indicated by SEQ ID NO: 1. Therefore, it became clear that peptides having the amino acid sequence indicated by SEQ ID NO: 1 are DRB3*01:01-binding peptides and DRB1*03:01-binding peptides, and that the portion of human AChRα1 having the amino acid sequence indicated by SEQ ID NO: 1 is an epitope for DRB3*01:01 and DRB1*03:01.

Additionally, from these results, the peptide indicated by SEQ ID NO: 16 (SEQ ID NO: 2) was observed in common in the samples from subjects in whom the HLA-DRB3 genotype was 01:01. Therefore, it became clear that peptides having the amino acid sequence indicated by SEQ ID NO: 2 are DRB3*01:01-binding peptides, and that the portion of human AChRα1 having the amino acid sequence indicated by SEQ ID NO: 2 is an epitope for DRB3*01:01.

Additionally, from these results, the peptides indicated by SEQ ID NOs: 22-32 were observed in common in the samples from subjects in whom the HLA-DRB3 genotype was 01:01, and they shared the amino acid sequence indicated by SEQ ID NO: 4. Therefore, it became clear that peptides having the amino acid sequence indicated by SEQ ID NO: 4 are DRB3*01:01-binding peptides, and that the portion of human AChRα1 having the amino acid sequence indicated by SEQ ID NO: 4 is an epitope for DRB3*01:01.

From these results, the peptides indicated by SEQ ID NOs: 33-35 were observed in common in the samples from subjects in whom the HLA-DRB3 genotype was 01:01 and the samples from subjects in whom the HLA-DRB1 genotype was 03:01, and they shared the amino acid sequence indicated by SEQ ID NO: 5. Therefore, it became clear that peptides having the amino acid sequence indicated by SEQ ID NO: 5 are DRB3*01:01-binding peptides and DRB1*03:01-binding peptides, and that the portion of human AChRα1 having the amino acid sequence indicated by SEQ ID NO: 5 is an epitope for DRB3*01:01 and DRB1*03:01.

### [Example 2: Specific T cell inducibility evaluation test using peripheral blood mononuclear cells (PBMCs) from healthy donors]

The peptides containing the epitopes discovered in Example 1 were studied regarding whether they have the function of inducing specific T cells from PBMCs from healthy donors.

### <Preparation of culture media>

Human AB-type serum (GeminiBio, Cat: 100-512) and fetal bovine serum (Nichirei, Cat: 175012) were subjected to inactivation treatments for 30 minutes at 56 °C, then filtered with a 0.2 µm filter and used. AIM-V (Gibco, Cat: 12055-091) containing 5% human AB-type serum was used as the culture medium for the PBMCs. The cells were cultured under 37 °C, 5% CO₂, water vapor saturated conditions. D-PBS(-) containing 2% fetal bovine serum was used as a Staining Buffer.

### <List of PBMCs from healthy donors>

The HLA alleles and evaluation peptides for the PBMCs from healthy donors used for the study in the present example are as indicated in Table 6. Six inducibility evaluation tests were performed for each peptide. The PBMCs were purchased from Precision for Medicine or STEMCELL Technologies. The boldface italics denote predicted restriction alleles of the peptides comprising the amino acid sequences according to SEQ ID NOs: 3, 16, 35, and 17, and the underlining denotes predicted restriction alleles of the peptides comprising the amino acid sequences according to SEQ ID NO: 17. The specimen indicated by the asterisk in Table 6 was evaluated in two independent tests.

**[Table 6]**

| PBMC Lot | DRB1* | | DRB3/4/5 | | DQB1* | | DPB1 * | | SEQ ID NO | | | |
|---|---|---|---|---|---|---|---|---|---|---|---|---|
| | | | | | | | | | 3 | 16 | 17 | 35 |
| 220781001C | 03:01 | 13:02 | 3*02:02 | - | 02:01 | 06:02 | 01:01 | 85:01 | ○ | | | ○ |
| 220882301C | 03:01 | - | ***3*01:01*** | - | 02:01 | - | 01:01 | - | ○ | ○ | ○ | ○ |
| 2211415004 | 03:01 | 01:01 | ***3*01:01*** | - | 02:01 | 05:01 | 04:01 | - | | | ○ | |
| 221170801C | 03:01 | - | ***3*01:01*** | - | 02:01 | - | 01:01 | 02:01 | ○ | ○ | ○ | ○ |
| 2011413006 | 03:01 | 03:02 | ***3*01:01*** | - | 02:01 | 04:02 | 01:01 | 15:01 | ○ | ○ 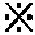 | ○ | ○ |
| 2209413009 | 03:01 | 14:01 | ***3*01:01*** | 3*02:02 | 02:01 | 05:03 | 02:01 | 04:02 | | | | |
| 2103402009 | 03:01 | 08:01 | ***3*01:01*** | - | 02:01 | 04:02 | 01:01 | 02:01 | ○ | ○ | | ○ |
| 2105411004 | 03:01 | 04:03 | ***3*01:01*** | 4*01:01 | 02:01 | 03:05 | 03:01 | 04:01 | ○ | ○ | | ○ |
| 202109977 | 03:01 | 04:01 | ***3*01:01*** | 4*01:03 | 02:01 | 03:01 | 01:01 | 04:01 | | | ○ | |
| 202145571 | 03:01 | - | ***3*01:01*** | - | 02:01 | - | 01:01 | 04:01 | | | ○ | |

### <List of evaluated peptides>

The evaluated peptides used for the study are as indicated in Table 7. These peptides were dissolved in DMSO (ATCC, Cat: 4-X) so as to be at a concentration of 20 mg/mL.

**[Table 7]**

| SEQ ID NO | Amino acid position in SEQ ID NO: 37 | Amino acid length | Sequence (N terminus → C terminus) | Synthetic manufacturer |
|---|---|---|---|---|
| 3 | 98-117 | 20 | GDFAIVKFTKVLLQYTGHIT | Eurofins genomics |
| 16 | 90-104 | 15 | LVLYNNADGDFAIVK | |
| 35 | 145-161 | 17 | KLGTWTYDGSVVAINPE | |
| 17 | 311-327 | 17 | WVRKVFIDTIPNIMFFS | GeneScript or Eurofins genomics |

### <Induction of peptide-specific T cells using PBMCs from healthy donors>

PBMCs that were preserved in a liquid nitrogen vapor phase were extracted and quickly thawed in a 37 °C hot water bath. The cells were washed once with AIM-V, resuspended in AIM-V containing 5% human AB-type serum (hereinafter, complete medium), and used to inoculate a 24-well plate with 1.5 × 10⁶ cells/well. A peptide was added such that the final concentration was 20 µg/mL and IL-7 (PeproTech, Cat: AF-200-07-50ug) was added such that the final concentration was 10 ng/mL, and culturing was initiated (Day 0, total culture medium amount: 2 mL/well).

After 1 week elapsed since initiating the culture, PBMCs from the same donors as the PBMCs being cultured were thawed in the same manner as on Day 0, washed once with AIM-V, and adjusted to 3 × 10⁶ cells /mL or less, and the same peptides as those used for induction on Day 0 were added at a concentration of 20 µg/mL and the cells were cultured for 2 hours. Furthermore, a mitomycin C solution was added such that the final concentration was 50 µg/mL, and the cells were further cultured for 45 minutes. Thereafter, the cells were washed three times with AIM-V to obtain antigen-presenting cells. The cells that were cultured for 1 week from Day 0 were recovered and used to inoculate a new 24-well plate within a range of 1 to 1.5 × 10⁶ cells /well. After inoculation with the same number of the prepared antigen-presenting cells, IL-7 was added such that the final concentration was 10 ng/mL and the culturing was resumed (Day 7, total culture medium amount: 2 mL/well). After 2 days, half of the culture solution was exchanged with a complete culture medium containing 40 U/mL of IL-2, and culturing was continued for 1 week while exchanging half of the culture solution every other day with the complete culture medium containing 20 U/mL of IL-2. At this time, when the cells became confluent, 2 mL/well of the culture solutions were well suspended, 1 mL thereof was used to inoculate a new well, and a complete culture medium containing 20 U/mL of IL-2 was added to each well such that the amount was 1 mL/well (hereinafter, passaging work). The cultured cells were recovered on Day 14, and used for the <Intracellular cytokine staining (ICS)> and the <IFN-γ producibility evaluation according to ELISA> mentioned below.

### <Intracellular cytokine staining (ICS)>

The recovered culture cells were used to inoculate 2 to 6 wells in a 96-well round-bottomed plate with 2.0 × 10⁶ each, the peptides used for induction were added at a concentration of 20 µg/mL to half of the wells, the solvent DMSO was added at a concentration of 0.1% to the other half, and the cells were cultured for 2 hours. Brefeldin A (BioLegend, Cat: 420601) and Monensin (BioLegend, Cat: 420701) were added such that the final concentration was 1 X, and the cells were further cultured for 4 hours. The cells were recovered, human CD3 antibody (BioLegend, Cat: 300440) labeled with FITC CD8a antibody (BioLegend, Cat: 100733) labeled with PerCP/Cy5.5, CD4 antibody (BioLegend, Cat: 100526) labeled with APC, and LIVE/DEAD Fixable Far Red Dead reagent (Invitrogen, Cat: L34973) diluted 500 times were added in Staining Buffer containing Fc Block (Miltenyi Biotec, Cat: 130-059-901) reagent, and reactions were allowed to take place for 15 minutes at 4 °C. Cytofix Cytoperm Fix/Perm Solution (Becton Dickinson, Cat: 554714) was added and the cells were treated for 20 minutes at 4 °C. The cells were washed with Perm./Wash Buffer (a 10X solution included in the kit (Cat: 554714) and diluted 10-fold with distilled water), human IFN-γ antibody (BioLegend, Cat: 502509) labeled with PE was added, a reaction was allowed to take place for 30 minutes at 37 °C, and following a wash with Perm./Wash Buffer, the cells were washed with Staining Buffer and analyzed by means of FACS.

### <IFN-γ producibility evaluation by ELISA>

The recovered culture cells were used to inoculate 6 wells in a 96-well round-bottomed plate with 1.0 × 10⁵ each, the peptides used for induction were added at a concentration of 20 µg/mL to half of the wells, the solvent DMSO was added at a concentration of 0.1% to the other half, and the cells were cultured for 16 hours. The culture supernatants were recovered and after appropriate dilution with Assay Diluent, were subjected to ELISA analysis. The IFN-γ concentration was measured by using a BD OptEIA ELISA Set (human IFN-g, Becton Dickinson, Cat: 555142) in accordance with attached documentation. In the case in which the absorbance was lower than that of a blank well, the IFN-γ concentration was treated as being 0.

### <Results>

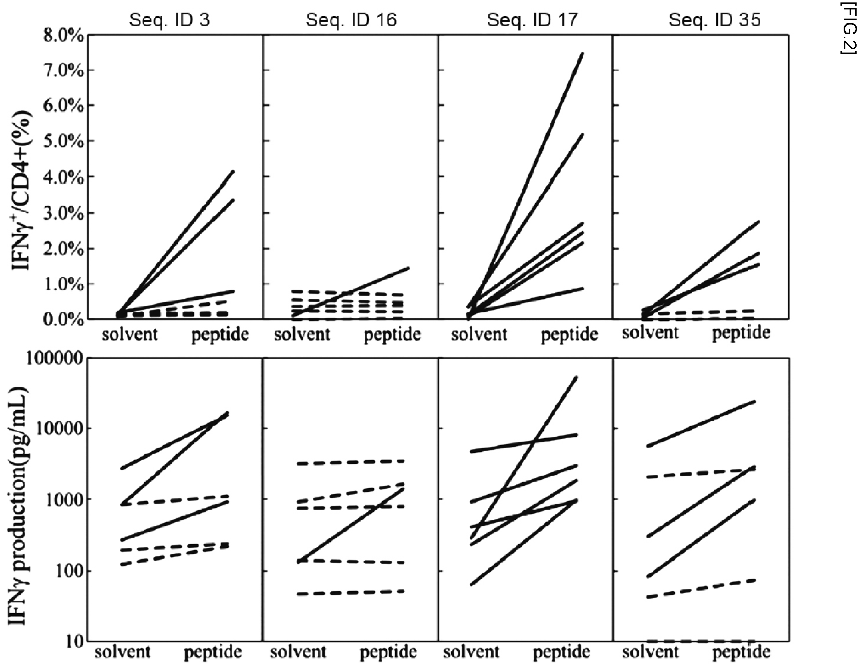

FIG. 2 is a diagram indicating the results of the ICS (upper section) and the ELISA (lower section) when using PBMCs from healthy donors to perform six independent specific T cell inducibility evaluation tests on the respective peptides. From the ICS results and the ELISA results, the specimens clearly having peptide-specific T cell induction were indicated by solid lines, and other specimens were indicated by dotted lines. Peptide-specific T cell induction was able to be observed in one out of the six examples for SEQ ID NO: 16, in three out of the six examples for SEQ ID NOs: 3 and 35, and in all of the six examples for SEQ ID NO: 17. It was made clear that there are helper T cells having T cell receptors (TCRs) recognizing HLA-peptide complexes in healthy donors. In particular, SEQ ID NO: 17, from the ICS results also, was found to have a high frequency of IFN-γ-producing CD4⁺ T cells and to have high potential when stimulated with peptides relative to a solvent control group (solvent = DMSO).

### [Example 3: Specific T cell inducibility evaluation test using peripheral blood mononuclear cells (PBMCs) from MG patients]

The peptide indicated by SEQ ID NO: 17, in which peptide-specific T cell induction was able to be confirmed in all examples in Example 2, was studied using PBMCs from MG in order to check whether the present peptide-specific Th1 cells are present in PBMCs from MG patients.

### <List of PBMCs from myasthenia gravis patients>

The HLA alleles of PBMCs from myasthenia gravis patients used in the study are as indicated in Table 8. The evaluated peptides were only peptides comprising the amino acid sequence according to SEQ ID NO: 17. The PBMCs were all purchased from Eolas Bio. The boldface italics denote predicted restriction alleles of SEQ ID NO: 17.

**[Table 8]**

| MG patient PBMC Lot | DRB1* | | DRB3/4/5 | | DQB1* | | DPB1 | |
|---|---|---|---|---|---|---|---|---|
| 145-KM1186 | 03:01 | 07:01 | ***3*01:01*** | 4*01:03 | 02:01 | 02:02 | 03:01 | 04:01 |
| 145-KM1187 | 01:01 | 13:01 | ***3*01:01*** | - | 05:01 | 06:03 | 02:01 | 04:02 |
| 281-4551-MG-2 | 11:04 | 13:01 | ***3*01:01*** | 3*02:02 | 06:03 | - | 04:01 | - |
| 281-4551-MG-9 | 13:01 | 15:01 | ***3*01.01*** | 5*01:01 | 06:02 | 06:03 | 02:01 | 04:01 |

### <Induction of peptide-specific T cells using PBMCs from MG patients>

T cells were induced basically by the same method as that in <Induction of peptide-specific T cells using PBMCs from healthy donors> in Example 2. However, since the purpose was to amplify memory T cells or effector T cells in the patient PBMCs, peptides and IL-7 were added on Day 0, the culture medium exchange with the complete culture medium containing IL-2 and the passaging work were started two days later, and the cells were provided for ELISA analysis on Day 7. The ELISA analysis was performed by the same method as that in <IFN-γ producibility evaluation by ELISA> in Example 2.

### <Results>

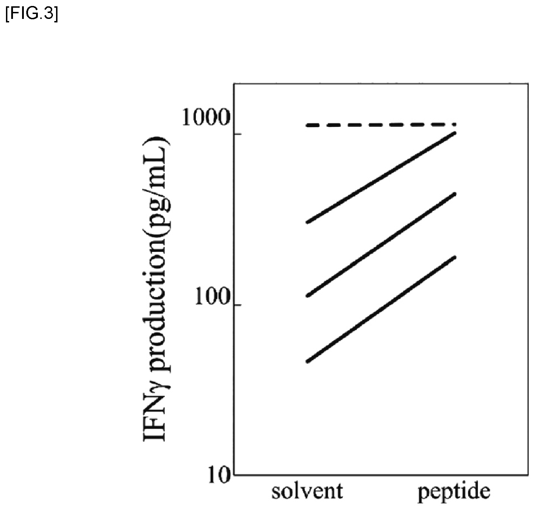

FIG. 3 is a diagram indicating the results of ELISA analysis in which five PBMC specimens from MG patients having DRB3*01:01, which is the predicted restriction allele of SEQ ID NO: 17, were used to perform an evaluation regarding the presence or absence of Th1 cells specific to SEQ ID NO: 17. As a result thereof, the presence of Th1 cells specific to SEQ ID NO: 17 was suggested for three out of four specimens. From the above results, Th1 cells specific to SEQ ID NO: 17 were present in DRB3*01:01-positive MG patients, suggesting their pathogenic potential.

### [Example 4: Study of possibility of further restrictiveness to other alleles]

In the ICS results in Example 2, the rate of Th1 cells specific to SEQ ID NO: 17 (the difference in the IFNγ-positive CD4⁺ T cells between when stimulated with peptides and when stimulated with DMSO) was 0.70% to 7.47% (median value: 2.3%), which is high. A healthy person can be considered not to have undergone Priming by endogenous antigens, and normally, Naive T cells will take 2 to 3 days after antigen stimulation to differentiate into Th1 cells. Thereafter, upon receiving additional stimulation, they will undergo clone amplification in the presence of IL-2. The doubling time of activated T cells is considered to be approximately 6 to 8 hours, and in the five days from Day 9 on which IL-2 is added until Day 14 on which the cells are recovered, a single cell will be amplified to approximately one million. Meanwhile, the diversity of TCRs is considered to be from 10⁸ to 10⁹, and if there is a single Naive T cell that recognizes a complex of HLA and a peptide, the presence ratio thereof is 10⁻⁹ to 10⁻⁸%, which can be considered to become approximately 0.1 to 1% upon undergoing the above-mentioned clone amplification. Since the experimental values obtained in Example 2 were higher than this theoretical value, it was suspected that SEQ ID NO: 17 may have additional restriction to other alleles. Therefore, the inducibility of Th1 cells specific to SEQ ID NO: 17 was evaluated by using PBMCs from healthy donors having HLA-DRB1*09:01, which is reported to be associated with MG in Asians, and not having DRB1*03:01 and DRB3*01:01.

### <List of PBMCs from healthy donors>

The HLA alleles in PBMCs from healthy donors used in the study in Example 4 are as shown in Table 9. The evaluated peptides were only peptides comprising the amino acid sequence according to SEQ ID NO: 17. The boldface italics denote alleles reported to be associated with the onset of myasthenia gravis in Asians.

**[Table 9]**

| PBMC Lot | DRB1* | | DRB3/4/5 | | DQB1* | | DPB1* | |
|---|---|---|---|---|---|---|---|---|
| 220781001G | 07:01 | ***09:01*** | 4*01:01 | - | 02:01 | 03:03 | 04:01 | 05:01 |
| 220882301G | ***09:01*** | 12:02 | 3*03:01 | 4*01:01 | 02:01 | 03:01 | 05:01 | 85:01 |
| 2211415004 | ***09:01*** | 11:01 | 3*02:02 | 4*01:01 | 03:01 | 03:03 | 05:01 | - |
| 221170801C | ***09:01*** | 15:01 | 4*01:01 | 5*01:01 | 03:03 | 06:02 | 03:01 | 04:01 |
| 2011413006 | ***09:01*** | 11:02 | 3*02:02 | 4*01:01 | 02:01 | 03:01 | 01:01 | 02:01 |
| 2209413009 | ***09:01*** | 15:01 | 4*01:03 | 5*01:01 | 03:03 | 06:02 | 04:01 | 06:01 |
| 2103402009 | ***09:01*** | 12:02 | 3*03:01 | 4*01:01 | 02:01 | 03:01 | 05:01 | 85:01 |
| 2105411004 | 07:01 | ***09:01*** | 4*01:01 | - | 02:01 | 03:03 | 04:01 | 05:01 |
| 202109977 | 04:04 | ***09:01*** | 4*01:01 | - | 03:02 | 03:03 | 03:01 | 04:02 |

### <Results>

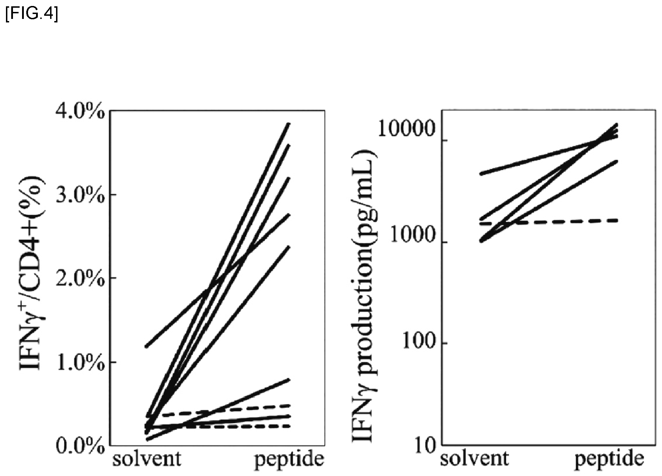

FIG. 4 is a diagram indicating the results of ICS (left side) and ELISA (right side) in which inducibility of Th1 cells specific to SEQ ID NO: 17 was evaluated using PBMCs from healthy donors having HLA-DRB1*09:01, which is reported to be associated with MG in Asians, and not having DRB1*03:01 and DRB3*01:01. As indicated in FIG. 4, Th1 cells specific to SEQ ID NO: 17 were induced in seven specimens out of nine specimens provided for ICS analysis (left side, solid lines). When five specimens (four specimens in which Th1 cells specific to SEQ ID NO: 17 were induced and one specimen in which they were not induced) out of the nine specimens provided for ICS analysis were then subjected to ELISA analysis, the production of IFNγ from the Th1 cells specific to SEQ ID NO: 17 was similarly detected in the four specimens in which induction was observed in ICS (right side, solid lines). The above results demonstrated that SEQ ID NO: 17 is restricted not only by the restriction alleles (DRB1*03:01 and DRB3*01:01) that were initially contemplated, but also by HLA-DRB1*09:01.

## Claims

1. A therapeutic agent or a preventive agent for an autoimmune disease, comprising at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

2. An activating agent for regulatory T cells, comprising at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

3. An activity inhibitory agent for effector T cells, comprising at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

4. An activation inhibitory agent for B cells, comprising at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

5. A suppressive agent for humoral immunity, comprising at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

6. A treatment method or a prevention method for an autoimmune disease, wherein the method includes administering, to a subject in need thereof, at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

7. At least one polypeptide for use in treating or preventing an autoimmune disease, wherein the polypeptide is selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

8. Use of at least one polypeptide in production of a therapeutic agent, a preventive agent, a composition for treatment, or a composition for prevention of an autoimmune disease, wherein the polypeptide is selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

9. A diagnostic agent for an autoimmune disease, comprising a complex comprising a polypeptide, a human leukocyte antigen, and a labeling dye, wherein the polypeptide is at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 5.

10. A kit for diagnosing an autoimmune disease, comprising at least one polypeptide and a human leukocyte antigen that binds to the at least one polypeptide, wherein the at least one polypeptide is selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 4.

11. The kit according to claim 10, further comprising a labeling dye that binds to the human leukocyte antigen.

12. A method for aiding in diagnosis of an autoimmune disease, wherein the method includes detecting T cells having antigen specificity to at least one polypeptide selected from the group consisting of:
(D) one or more polypeptides having a full length of at least 7 residues and at most 17 residues, including an amino acid sequence having 90% or higher sequence identity with an amino acid sequence according to SEQ ID NO: 4;
(A) one or more polypeptides having a full length of at least 8 residues and at most 20 residues, including an amino acid sequence having 90% or higher sequence identity with an amino acid sequence according to SEQ ID NO: 1;
(B) one or more polypeptides having a full length of at least 12 residues and at most 21 residues, including an amino acid sequence having 90% or higher sequence identity with an amino acid sequence according to SEQ ID NO: 2; and
(E) one or more polypeptides having a full length of at least 10 residues and at most 19 residues, including an amino acid sequence having 90% or higher sequence identity with an amino acid sequence according to SEQ ID NO: 4.
